(19) Europäisches Patentamt
European Patent Office
Office européen des brevets

(11) Veröffentlichungsnummer: **0 377 903 A2**

# (12) EUROPÄISCHE PATENTANMELDUNG

(21) Anmeldenummer: **89124122.6**

(22) Anmeldetag: **28.12.89**

(51) Int. Cl.5: **C07D 253/075, A01N 43/76, A01N 43/78, A01N 43/707, C07D 417/12, C07D 413/12, C07D 401/12, C07D 403/12, //C07D213/643**

(30) Priorität: **09.01.89 DE 3900373**

(43) Veröffentlichungstag der Anmeldung: **18.07.90 Patentblatt 90/29**

(84) Benannte Vertragsstaaten: **BE CH DE ES FR GB IT LI NL**

(71) Anmelder: **BAYER AG**

**D-5090 Leverkusen 1 Bayerwerk(DE)**

(72) Erfinder: **Lindner, Werner, Dr.**
**Märchenstrasse 39**
**D-5000 Köln 80(DE)**
Erfinder: **Haberkorn, Axel, Prof. Dr.**
**Fuhlrottstrasse 99**
**D-5600 Wuppertal 1(DE)**

(54) **Substituierte Hexahydro-1,2,4-triazindione, Verfahren zu ihrer Herstellung, Zwischenprodukte dafür und ihre Verwendung.**

(57) Die vorliegende Erfindung betrifft:
1. Neue substituierte Hexahydro-1,2,4-triazindione der allgemeinen Formel I

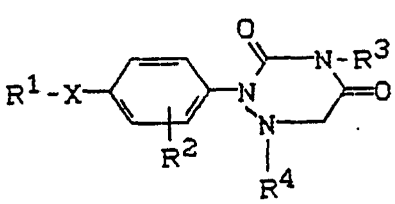

I

in welcher
$R^1$ für über Kohlenstoff gebundene heteroaromatische Reste steht, die gegebenenfalls substituiert sind,
X für O, S, SO, $SO_2$ oder $-CR^5(CN)-$ steht,
$R^2$ für einen oder mehrere, gleiche oder verschiedene Reste der Gruppe Wasserstoff, Halogen, Nitro, Alkyl, Alkoxy, Halogenalkyl, Halogenalkoxy, Alkylthio, Halogenalkylthio,
$R^3$ und $R^4$ unabhängig voneinander für Wasserstoff, gegebenenfalls substituiertes Alkyl, Alkenyl, Alkinyl, Aralkyl stehen,
$R^5$ für Wasserstoff oder Alkyl steht,
ausgenommen Verbindungen, in denen X für $-CR^5(CN)-$ und $R^1$ für Thienyl steht,
Verfahren zu ihrer Herstellung, Zwischenprodukte zur Durchführung dieser Verfahren und deren Herstellung, sowie ihre Verwendung zur Bekämpfung parasitärer Protozoen, insbesondere Coccidien sowie Fischparasiten.

EP 0 377 903 A2

**Substituierte Hexahydro-1,2,4-triazindione, Verfahren zu ihrer Herstellung, Zwischenprodukte dafür und ihre Verwendung**

Die vorliegende Erfindung betrifft neue substituierte Hexahydro-1,2,4-triazindione, Verfahren zu ihrer Herstellung, Zwischenprodukte zur Durchführung dieser Verfahren, sowie ihre Verwendung zur Bekämpfung parasitärer Protozoen und insbesondere Coccidien sowie Fischparasiten.

Die Verwendung von substituierten 1,2,4-Triazindionen zur Bekämpfung von Coccidien ist bekannt. Die Wirkung dieser Verbindungen befriedigt jedoch nicht in jedem Fall.

Die vorliegende Erfindung betrifft:

1. Neue substituierte Hexahydro-1,2,4-triazindione der allgemeinen Formel I

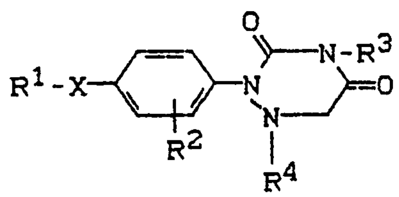

I

in welcher
$R^1$ für über Kohlenstoff gebundene heteroaromatische Reste steht, die gegebenenfalls substituiert sind,
X für O, S, SO, $SO_2$ oder $-CR^5(CN)-$ steht,
$R^2$ für einen oder mehrere, gleiche oder verschiedene Reste der Gruppe Wasserstoff, Halogen, Nitro, Alkyl, Alkoxy, Halogenalkyl, Halogenalkoxy, Alkylthio, Halogenalkylthio,
$R^3$ und $R^4$ unabhängig voneinander für Wasserstoff, gegebenenfalls substituiertes Alkyl, Alkenyl, Alkinyl, Aralkyl stehen,
$R^5$ für Wasserstoff oder Alkyl steht,
ausgenommen Verbindungen, in denen X für $-CR^5(CN)-$und $R^1$ für Thienyl steht.

2. Verfahren zur Herstellung von substituierten Hexahydro-1,2,4-triazindionen der allgemeinen Formel I

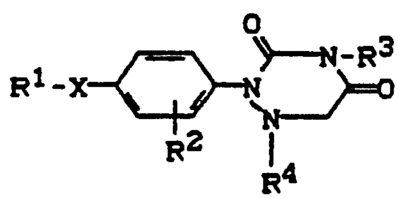

I

in welcher
$R^1$ für über Kohlenstoff gebundene heteroaromatische Reste steht, die gegebenenfalls substituiert sind,
X für O, S, SO, $SO_2$ oder $-CR^5(CN)-$ steht,
$R^2$ für Wasserstoff einen oder mehrere, gleiche oder verschiedene Reste der Gruppe Wasserstoff, Halogen, Nitro, Alkyl, Alkoxy, Halogenalkyl, Halogenalkoxy' Alkylthio, Halogenalkylthio,
$R^3$ und $R^4$ unabhängig voneinander für Wasserstoff, gegebenenfalls substituiertes Alkyl, Alkenyl, Alkinyl, Aralkyl stehen,
$R^5$ für Wasserstoff oder Alkyl steht,
ausgenommen Verbindungen, in denen X für $-CR^5(CN)-$und $R^1$ für Thienyl steht,

a) dadurch gekennzeichnet, daß man Verbindungen der Formel II

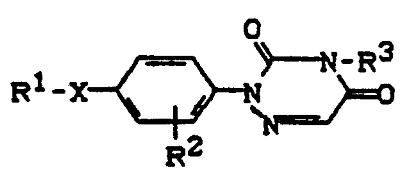

II

in welcher
X, $R^1$, $R^2$ und $R^3$ die oben angegebene Bedeutung haben,
hydriert oder

b) dadurch gekennzeichnet, daß man Verbindungen der Formel III

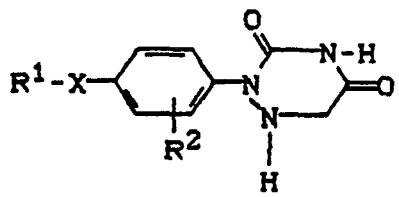

III

in welcher
$R^1$, $R^2$, X die oben angegebene Bedeutung haben
mit Verbindungen der Formel IV

$R^3$-B IV

in welcher
$R^3$ für gegebenenfalls substituiertes Alkyl, Alkenyl, Alkinyl, Aralkyl steht und
B für Halogen, -O-$SO_2$-Alkyl, -O-$SO_2$-Aryl, -O-$SO_2$-Halogenalkyl steht
umsetzt, oder

c) dadurch gekennzeichnet, daß man Verbindungen der Formel V

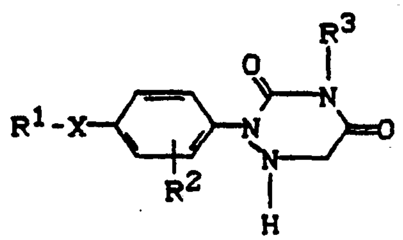

V

in welcher
$R^1$, $R^2$, $R^3$ und X die oben angegebene Bedeutung haben, $R^3$ aber nicht für Wasserstoff steht,
mit Verbindungen der Formel VI

$R^4$-B VI

in welcher
$R^4$ und B die oben angegebene Bedeutung haben,
umsetzt.

3. Neue Verbindungen der Formel II

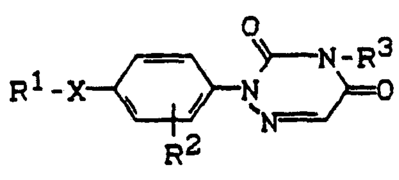

II

in welcher
X die in 1. angegebene Bedeutung hat,
$R^1$, $R^2$, $R^3$ die unter (1) angegebenen Bedeutungen besitzen, wobei $R^1$ nicht für Thiophen stehen darf, wenn X für -$CR^5$(CN) steht und nicht für Benzthiazol, Benzoxazol, Thiazol und Oxazol stehen darf, wenn X für O, S, SO oder $SO_2$ steht.

4. Verfahren zur Herstellung der neuen Verbindungen der Formel II gemäß (3) sowie bekannter Verbindungen der Formel II, dadurch gekennzeichnet,

a) daß man Verbindungen der Formel VII

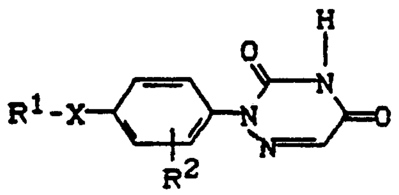

VII

in welcher
X, $R^1$ und $R^2$ die in (3) angegebene Bedeutung haben,
mit Verbindungen der Formel IV
$R^3$-B IV
in welcher
$R^3$ und B die in (2) angegebenen Bedeutungen haben,
umsetzt, oder

b) daß man Verbindungen der Formel VIII

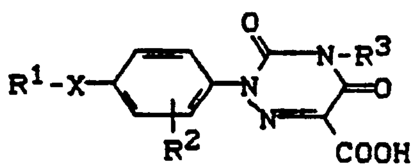

VIII

in welcher
X, $R^1$, $R^2$, $R^3$, $R^5$ die in (1) angegebenen Bedeutungen haben,
durch Erhitzen decarboxyliert oder

c) daß man Verbindungen der Formel IX

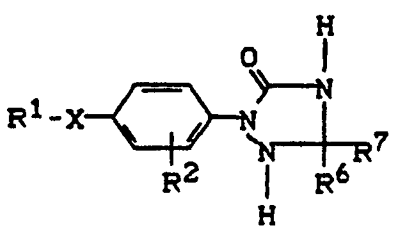

IX

in welcher
X für O, S, $-CR^5(CN)-$ steht,
$R^1$, $R^2$ die in (1) angegebene Bedeutung haben,
$R^6$ und $R^7$ für gegebenenfalls substituiertes Alkyl stehen,
mit Glyoxylsäure der Formel X
CHO-COOH X
in Gegenwart von anorganischen oder organischen Säuren umsetzt, oder

d) daß man Verbindungen der Formel XI

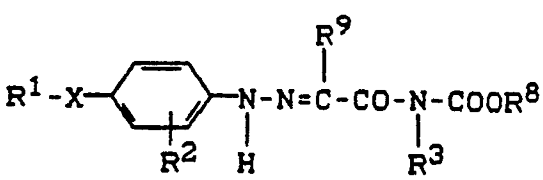

XI

in welcher
X für O, S oder $-CR^5(CN)-$ steht,
$R^1$, $R^2$, $R^3$, $R^5$ die in (1) angegebene Bedeutung haben, und
$R^8$ für Alkyl oder gegebenenfalls substituiertes Aryl steht,

$R^9$ für H steht,
in Gegenwart von Basen erhitzt,
e) daß man Verbindungen der Formel XVII

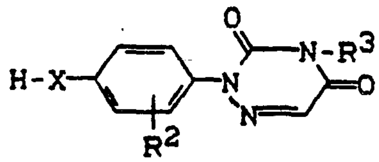

XVII

in welcher
X für O oder S steht,
$R^2$, $R^3$ die oben angegebene Bedeutung haben,
mit Verbindungen der Formel XVIII $R^1$-A XVIII
in welcher
$R^1$ die oben angegebene Bedeutung hat und
A für die Reste Halogen, $O-SO_2$-Alkyl, $-O-SO_2$-Halogenalkyl, $-O-SO_2$-Aryl, -S-Alkyl, $-SO_2$-Alkyl oder $SO_2$-Halogenalkyl steht, umsetzt.

5. Neue Verbindungen der Formel VIII

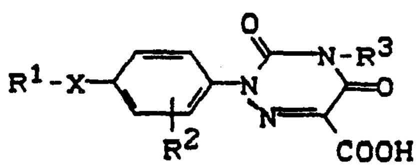

VIII

in welcher
X, $R^1$, $R^2$, $R^3$ die in (1) angegebene Bedeutung haben.

6. Verfahren zur Herstellung der neuen Verbindungen der Formel VIII gemäß (5), dadurch gekennzeichnet, daß man Verbindungen der Formel XII

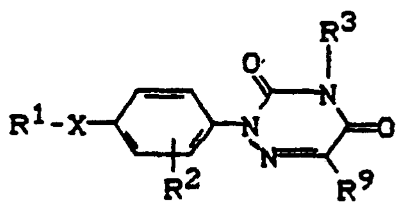

XII

in welcher
X, $R^1$, $R^2$, $R^3$ die in (1) angegebene Bedeutung haben,
$R^8$ für Alkyl oder Aryl steht,
$R^9$ für CN oder den Rest $-CO-N(R^3)-COOR^8$ steht,
in Gegenwart von wäßrigen Mineralsäuren erhitzt.

7. Neue Verbindungen der Formel XII

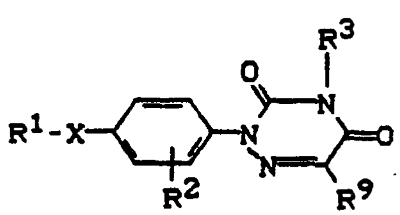

XII

in welcher
X, $R^1$, $R^2$, $R^3$, $R^8$ die in (6) angegebene Bedeutung haben, und

$R^9$ für CN oder den Rest -CO-N($R^3$)-COOR$^8$ steht.

8. Verfahren zur Herstellung der neuen Verbindungen der Formel XII gemäß (7), dadurch gekennzeichnet, daß man Verbindungen der Formel XI

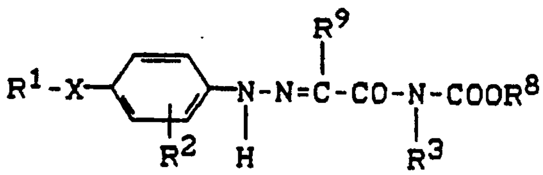

XI

in welcher
X, $R^1$, $R^2$, $R^3$, $R^8$, $R^9$ die in (7) angegebene Bedeutung haben,
in Gegenwart von Basen erhitzt.

9. Neue Verbindungen der Formel XI

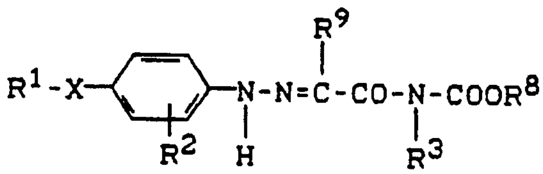

XI

in welcher
X, $R^1$, $R^2$, $R^3$, $R^8$, $R^9$ die in (7) angegebene Bedeutung haben und $R^9$ zusätzlich für Wasserstoff stehen kann.

10. Verfahren zur Herstellung der neuen Verbindungen der Formel XI, dadurch gekennzeichnet, daß man

a) Verbindungen der Formel XIII

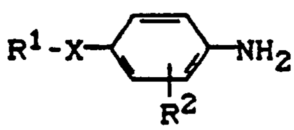

XIII

in welcher
X, $R^1$, $R^2$ die in (1) angegebene Bedeutung haben,
in an sich bekannter Weise diazotiert und anschließend mit Verbindungen der Formel XIV

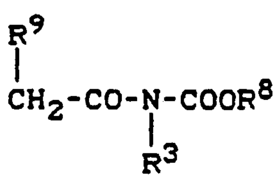

XIV

in welcher
$R^3$, $R^8$, $R^9$ die in (7) angegebene Bedeutung haben,
umsetzt, oder

b) indem man Verbindungen der Formel XV

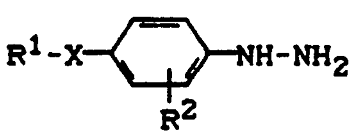

XV

in welcher

X, $R^1$, $R^2$ die in (1) angegebene Bedeutung haben,
zunächst mit Glyoxylsäure der Formel X, anschließend mit Thionylchlorid und dann mit Ethylurethan umsetzt.

11. Neue Verbindungen der Formel IX

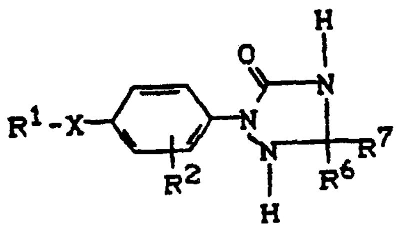

IX

in welcher
X, $R^1$, $R^2$ die in (1) angegebene Bedeutung haben,
$R^6$ und $R^7$ unabhängig voneinander für gegebenenfalls substituiertes Alkyl stehen.

12. Verfahren zur Herstellung der neuen Verbindungen der Formel IX, dadurch gekennzeichnet, daß man Verbindungen der Formel XV

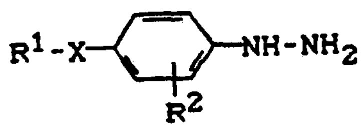

XV

in welcher
X, $R^1$, $R^2$ die in (1) angegebene Bedeutung haben,
zunächst mit Ketonen der Formel XVI

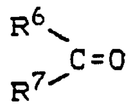

XVI

in welcher
$R^6$ und $R^7$ die oben angegebene Bedeutung haben,
und anschließend mit Alkalicyanaten umsetzt.

Die Verbindungen der Formel I sowie ihre Salze mit Basen oder Säuren sind hervorragend zur Bekämpfung parasitärer Protozoen und insbesondere von Coccidien sowie Fischparasiten geeignet.

Bevorzugte Verbindungen der Formel I sind Verbindungen in denen
$R^1$ für gegebenenfalls durch Halogen, Alkyl, Cyano, Nitro, Alkoxy, Alkylthio, Halogenalkyl, Halogenalkoxy, Halogenalkylthio, Halogenalkylsulfonyl, Halogenalkylsulfinyl, Amino, Alkylamino, Halogenalkylamino, Acylamino substituiertes Thiazolyl, Oxazolyl, Benzthiazolyl, Benzoxazolyl, Pyrimidinyl, Pyridinyl, Chinolinyl oder Chinoxalinyl steht,
X für O, S oder -CH(CN)- steht,
$R^2$ für Halogen oder $C_{1-6}$-Alkyl steht,
$R^3$ für Wasserstoff oder $C_1$-$C_4$-Alkyl, insbesondere Methyl, steht,
$R^4$ für Wasserstoff oder $C_{1-4}$-Alkyl steht.

Besonders bevorzugt sind Verbindungen der Formel I in welcher
X für O steht,
$R^1$ für Pyridinyl oder Benzthiazolyl, die gegebenenfalls durch $C_{1-4}$-Alkyl, insbesondere Methyl, $C_{1-4}$-Halogenalkyl insbesondere Trifluormethyl, Halogen, insbesondere Chlor, Brom, Fluor, Nitro, CN, $C_{1-4}$-Alkoxy insbesondere Methoxy, $C_{1-4}$-Halogenalkoxy insbesondere Trifluormethoxy, $C_{1-4}$-Alkylthio insbesondere Methylthio, $C_{1-4}$-Halogenalkylthio insbesondere Trifluormethylthio, $C_{11-4}$ Halogenalkylsulfonyl, insbesondere Trifluormethylsulfonyl, $C_{1-4}$-Halogenalkylsulfinyl, insbesondere Trifluormethylsulfinyl, Amino, $C_{1-4}$-Alkylamino, $C_{14}$-Halogenalkylamino, Acylamino insbesondere Acetylamino substituiert sind, steht,

$R^2$ für einen oder mehrere Reste der Gruppe Wasserstoff oder Halogen insbesondere Chlor, Brom, $C_{1-4}$-Alkyl insbesondere Methyl, 1-5-Halogen($C_{1-4}$)-alkyl, insbesondere Trifluormethyl steht,
$R^3$ für Wasserstoff oder Methyl steht,
$R^4$ für Wasserstoff steht.

Ganz besonders bevorzugt sind Verbindungen der Formel I
in welcher
X für O steht,
$R^1$ für gegebenenfalls durch Chlor oder Methyl oder Trifluormethyl, Trifluoromethylthio oder Trifluoromethoxy oder Fluor, substituiertes Benzthiazolyl oder Pyridinyl steht,
$R^2$ für einen oder mehrere Reste der Gruppe Wasserstoff, Methyl oder Chlor steht,
$R^3$ für Wasserstoff oder Methyl steht,
$R^4$ für Wasserstoff steht.

Als Einzelverbindungen seien genannt:

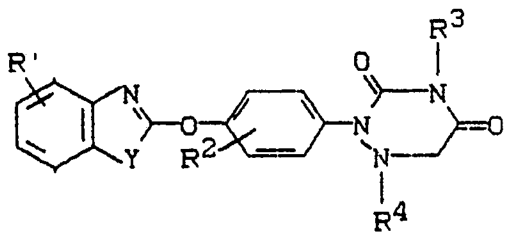

| Y | $R^2$ | $R^3$ | $R^4$ | R' |
|---|---|---|---|---|
| S | 3,5-$Cl_2$ | H | H | 6-Cl |
| S | 2,5-$Cl_2$ | H | H | 5,6-Cl |
| S | 3-$CH_3$ | H | H | 6-Cl |
| S | 3-$CH_3$ | H | H | 5,6-Cl |
| S | 3,5-$Cl_2$ | $CH_3$ | H | 6-Cl |
| S | 3,5-$Cl_2$ | $C_2H_5$ | H | 6-Cl |
| S | 3,5-$Cl_2$ | H | H | 6-$CF_3$ |
| S | 3-$CH_3$ | H | H | 6-$CF_3$ |
| S | 3,5-$Cl_2$ | H | H | 6-$SCF_3$ |
| S | 3,5-$Cl_2$ | H | H | 6-Br |
| S | 3,5-$Cl_2$ | H | H | 6-F |
| S | 3,5-$Cl_2$ | H | H | 6-$CH_3$ |
| S | 3,5-$Cl_2$ | H | H | 6-$OCF_3$ |

Weiterhin seien die folgenden Verbindungen genannt:

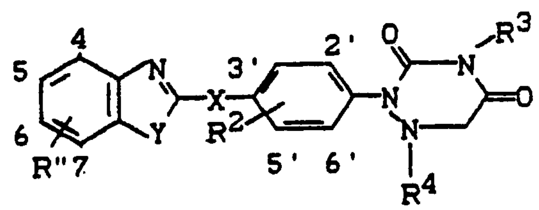

| Y | $R^2$ | X = O | $R^3$ | R'' | $R^4$ |
|---|---|---|---|---|---|
| S | H | | H | H | H |
| S | H | | H | 6-Cl | H |
| S | H | | H | 6-Br | H |
| S | H | | H | 6-F | H |
| S | H | | H | 6-$CH_3$ | H |
| S | H | | H | 6-$OCH_3$ | H |
| S | H | | H | 6-$NO_2$ | H |
| S | H | | H | 6-CN | H |
| S | H | | H | 6-$CF_3$ | H |
| S | H | | H | 6-$SCF_3$ | H |
| S | H | | H | 6-$OCF_3$ | H |
| S | H | | H | 5-Cl | 6-Cl |
| S | 3'-$CH_3$ | | H | H | H |
| S | 3'-$CH_3$ | | H | 6-Br | H |
| S | 3'-$CH_3$ | | H | 6-F | H |
| S | 3'-$CH_3$ | | H | 6-$CH_3$ | H |
| S | 3-$CH_3$ | | H | 6-$OCH_3$ | H |
| S | 3-$CH_3$ | | H | 6-$NO_2$ | H |
| S | 3-$CH_3$ | | H | 6-CN | H |
| S | 3-$CH_3$ | | H | 6-$SCF_3$ | H |
| S | 3-Cl | | H | H | H |
| S | 3-Cl | | H | 6-Cl | H |

| Y | $R^2$ X = O | $R^3$ | R" | $R^4$ |
|---|---|---|---|---|
| S | 3-Cl | H | 6-Br | H |
| S | 3-Cl | H | 6-F | H |
| S | 3'-Cl | H | 6-$CH_3$ | H |
| S | 3'-Cl | H | 6-$OCH_3$ | H |
| S | 3'-Cl | H | 6-$NO_2$ | H |
| S | 3'-Cl | H | 6-CN | H |
| S | 3'-Cl | H | 6-$CF_3$ | H |
| S | 3'-Cl | H | 6-$SCF_3$ | H |
| S | 3'-Cl | H | 6-$OCF_3$ | H |
| S | 3'-Cl | H | 5,6-Cl | H |
| S | 3',5'-$Cl_2$ | H | H | H |
| S | 3',5'-$Cl_2$ | H | 6-$OCH_3$ | H |
| S | 3',5'-$Cl_2$ | H | 6-$NO_2$ | H |
| S | 3',5'-$Cl_2$ | H | 6-CN | H |
| S | 3'-Cl, 5'-$CH_3$ | H | H | H |
| S | 3'-Cl, 5'-$CH_3$ | H | 6-Cl | H |
| S | 3'-Cl, 5'-$CH_3$ | H | 6-Br | H |
| S | 3'-Cl, 5'-$CH_3$ | H | 6-F | H |
| S | 3'-Cl, 5'-$CH_3$ | H | 6-$OCH_3$ | H |
| S | 3'-Cl, 5'-$CH_3$ | H | 6-CN | H |
| S | 3'-Cl, 5'-$CH_3$ | H | 6-CN | H |
| S | 3'-Cl, 5'-$CH_3$ | H | 6-$CF_3$ | H |
| S | 3'-Cl, 5'-$CH_3$ | H | 6-$SCF_3$ | H |
| S | 3'-Cl, 5'-$CH_3$ | H | 5,6-Cl | H |
| S | 3'-$CH_3$, 5'-$CH_3$ | H | 6-Cl | H |
| S | 3'-Cl, 5'-Cl | H | 6-$SO_2CF_3$ | H |
| S | 3'-Cl, 5'-Cl | H | 6-$SOCF_3$ | H |

| Y | $R^2$ X = O | $R^3$ | R" | $R^4$ |
|---|---|---|---|---|
| S | 3'-$CH_3$, 5'-$CH_3$ | H | 5,6-Cl | H |
| S | 3'-$CH_3$, 5-$CH_3$ | H | 5-Cl | H |
| S | 3'-Cl | H | 5-Cl | H |
| S | 3'-$CH_3$ | H | 5-Cl | H |
| S | 3'-Cl, 5'-$CH_3$ | H | 5-Cl | H |
| S | 3'-Cl, 5'-Cl | H | 5-Cl | H |
| S | 3'-Br | H | 6-Cl | H |
| S | 3'-Br, 5'-Br | H | 6-Cl | H |
| S | 3'-$CF_3$ | H | 6-Cl | H |
| S | 3'-$CF_3$, 5'-Cl | H | 6-Cl | H |
| O | 3'-$CH_3$ | H | 6-Cl | H |
| S | 3'-Cl, 5'-Cl | $CH_3$ | 6-Cl | H |
| S | 3'-$CH_3$ | -$C_2H_5$ | 5,6-Cl | H |
| O | 3,5-Cl | H | 6-Cl | H |
| O | 3,5-Cl | H | 5-Cl | H |
| S | 3'-Cl, 5'-Cl | H | 6-$SCF_3$ | H |

| Y | X | R2 | $R^3$ | R" | $R^4$ |
|---|---|---|---|---|---|
| S | S | H | H | 6-Cl | H |
| S | S | H | H | H | H |
| O | S | H | H | H | H |
| O | SO | H | H | H | H |
| O | $SO_2$ | H | H | H | H |
| O | S | 3,5-C12 | H | 6-Cl | H |
| O | S | 3,5-C12 | H | H | H |
| S | CHCN | H | H | H | H |
| S | CHCN | Cl | H | H | H |
| S | CHCN | 3,5-$Cl_2$ | H | H | H |
| S | $C(CH_3)CN$ | H | H | H | H |
| S | $C(CH_3)CN$ | H | $CH_3$ | H | H |

Weiter seien folgende Verbindungen der Formel I genannt:

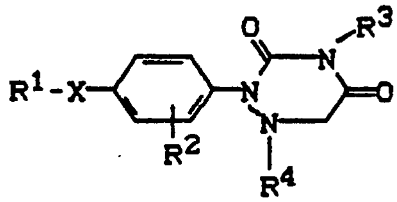

| $R^1$ | X | $R^2$ | $R^3$ | $R^4$ |
|---|---|---|---|---|
| 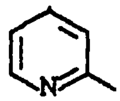 | CHCN | $3,5-Cl_2$ | H | H |
| 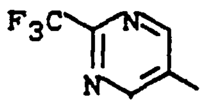 | O | $3,5-Cl_2$ | H | H |
| 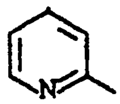 | O | $3,5-Cl_2$ | H | H |
| " | | $3-CH_3$ | H | H |
| 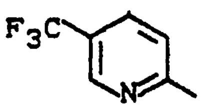 | O | $3,5-Cl_2$ | H | H |
| " | | $3-CH_3$ | H | H |
| 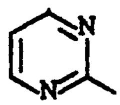 | O | $3,5-Cl_2$ | H | H |
| " | O | $3-CH_3$ | H | H |
| 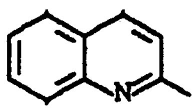 | O | $3,5-Cl_2$ | H | H |
| " | O | $3-CH_3$ | H | H |

| | | | | |
|---|---|---|---|---|
| 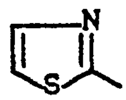 | O | 3,5-$Cl_2$ | H | H |
| " | O | 3,5-$(CH_3)_2$ | H | H |
| 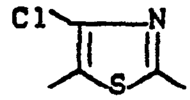 | O | 3,5-$Cl_2$ | H | H |
| " | O | 3-$CH_3$ | H | H |
| 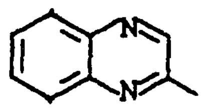 | O | 3,5-$Cl_2$ | H | H |
| 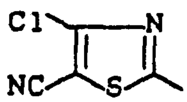 | O | 3,5-$Cl_2$ | H | H |
| " | O | 3-$CH_3$ | H | H |
| 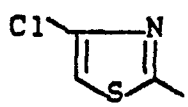 | O | 3,5-$Cl_2$ | H | H |
| 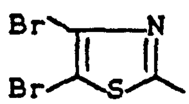 | O | 3,5-$Cl_2$ | H | H |
| " | O | 3,5-$(CH_3)_2$ | H | H |
| " | O | 3-$CH_3$ | H | H |

Nach Verfahren 2a) lassen sich sowohl die Verbindungen der Formel I als auch die Verbindungen der Formeln III und V herstellen.

Setzt man bei dem Verfahren 2a) als Verbindung der Formel II 2-(4-(2-Benzthiazolyloxy)-phenyl)-1,2,4-triazin-3,5-(2H,4H)dion ein, läßt sich das Verfahren durch folgendes Formelschema beschreiben:

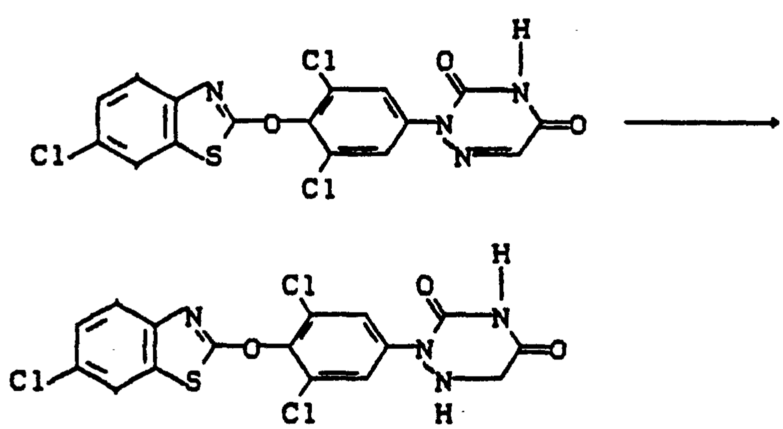

Die Verbindungen der Formel II sind zum Teil neu (vgl. 3 unten) und werden z.B. nach den in (4) angegebenen Verfahren erhalten.

Bevorzugt seien Verbindungen der Formel II genannt, in denen X, $R^1$, $R^2$, $R^3$ die bei den Verbindungen der Formel 1 genannten bevorzugten Bedeutungen haben.

Im einzelnen seien folgende Verbindungen der Formel II genannt:

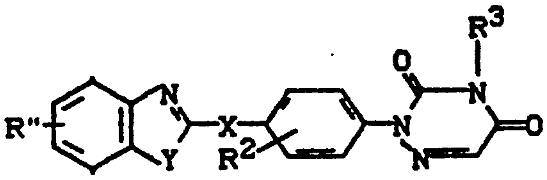

| Y | X | $R^2$ | $R^3$ | R" |
|---|---|---|---|---|
| S | CHCN | 3,5-Cl | H | H |

Weiter seien als Verbindungen der Formel II genannt:

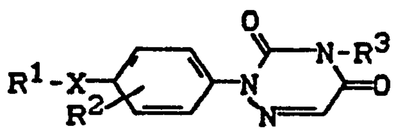

| $R^1$ | X | $R^2$ | $R^3$ |
|---|---|---|---|
| 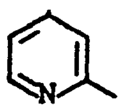 | -CH(CN)- | 3,5-$Cl_2$ | H |
| 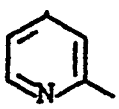 | O | 3,5-$Cl_2$ | H |
| " | O | 3-$CH_3$ | H |
| 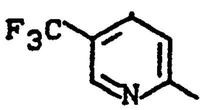 | O | 3,5-$Cl_2$ | H |
| 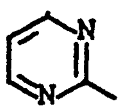 | O | 3,5-$Cl_2$<br>3-$CH_3$ | H<br>H |
| " | O | 3-$CH_3$ | H |
| 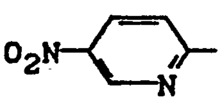 | O | 3,5-$Cl_2$ | H |
| 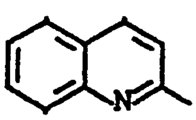 | O | 3,5-$Cl_2$ | H |
| 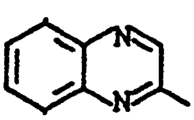 | O | 3,5-$Cl_2$ | H |

| $R^1$ | X | $R^2$ | $R^3$ |
|---|---|---|---|
| 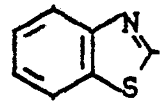 | -CH(CN)- | 3,5-$Cl_2$ | H |
| " | -$CH_3$CN | H | H |
| " | -$CH_3$CN | H | $CH_3$ |
| 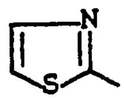 | O | 3,5-$Cl_2$ | H |
| " | O | 3,5-$(CH_3)_2$ | H |
| 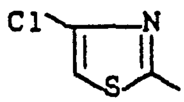 | O | 3,5-$Cl_2$ | H |
| " | | 3-$CH_3$ | H |
| 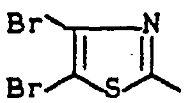 | O | 3,5-$Cl_2$ | H |
| " | O | 3,5-$(CH_3)_2$ | H |
| " | O | 3-$CH_3$ | H |

Das Verfahren 2a) wird durchgeführt, indem man eine Verbindung der Formel II in Gegenwart eines Reduktionsmittels und einer Säure erwärmt. Als Reduktionsmittel können Metalle wie z.B. Zink und Metallsalze wie z.B. Zinn(II)chlorid, Metallhydride wie Lithiumaluminiumhydrid und katalytisch angeregter Wasserstoff verwendet werden.

Als Säuren kommen verdünnte Mineralsäuren wie z.B. Salzsäure und organische Säuren wie z.B. Eisessig zum Einsatz. Die Reaktion kann gegebenenfalls in Gegenwart eines Verdünnungsmittels durchgeführt werden. Als Verdünnungsmittel können inerte organische Lösungsmittel verwendet werden. Hierzu gehören Kohlenwasserstoffe wie zB Toluol, Ether wie z.B. Dioxan, Ketone wie z.B. Aceton und Alkohole wie z.B. Ethanol. Die Reduktion erfolgt bei Temperaturen zwischen 80 und 120 °C bei Normaldruck oder erhöhtem Druck.

Nach Verfahren 2b lassen sich sowohl die Verbindungen der Formel I als auch die Verbindungen der Formel V herstellen.

Setzt man bei dem Verfahren 2b) als Verbindung der Formel III 2-[4-[6′-Chloro)-2′-benzothiazolyloxy]-3,5-dichlorphenyl]-hexahydro-1,2,4-triazin-3,5-dion und als Verbindung der Formel IV Methyliodid ein, läßt sich das Verfahren durch folgendes Schema beschreiben:

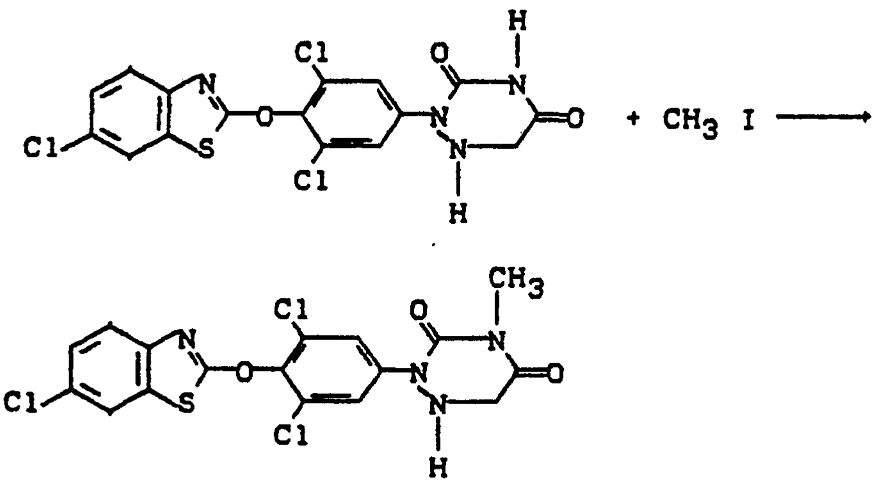

Die Verbindungen der Formel III werden durch Formel I umfasst und können wie bei Verfahren 2a) beschrieben hergestellt werden.

Die Verbindungen der Formel IV sind bekannt oder lassen sich nach bekannten Methoden darstellen.

Es werden bevorzugt Verbindungen der Formeln III und IV eingesetzt, in denen die Reste X, $R^1$, $R^2$, $R^3$ die bei den Verbindungen der Formel I angegebenen bevorzugten Bedeutungen besitzen.

Das Verfahren 2b) wird durchgeführt, indem man eine Verbindung der Formel III gegebenenfalls in Gegenwart einer Base und eines Verdünnungsmittels mit Verbindungen der Formel IV umsetzt. Als Verdünnungsmittel können alle inerten organischen Lösungsmittel eingesetzt werden.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Das Verfahren wird in Gegenwart von Basen durchgeführt. Als bevorzugte Basen seien genannt die Alkalihydroxide wie Natriumyhdroxid, Alkalialkoholate wie Natriummethylat oder Kaliumbutanolat, Metallhydride wie Natriumhydrid oder organische Basen wie 1,8-Diazabicyclo[5,40]-undec-7-en (DBU).

Das Verfahren wird durchgeführt bei Normaldruck und Temperaturen zwischen 20° und 140° C.

Die Reaktion wird durchgeführt, indem man äquimolare Mengen der Verbindung der Formel Ia und Base zusammengibt, dieses Gemisch mit einer äquimolaren Menge der Verbindung der Formel IV versetzt und auf die Reaktionstemperatur erhitzt.

Setzt man bei dem Verfahren 2c) als Verbindung der Formel V 2-[4-[6′-Chloro)-2′-benzthiazolyloxy]-3,5-dichlorophenyl]-4-methyl-hexahydro-1,2,4-triazin-3,5-dion ein und als Verbindung der Formel VI Ethyliodid, läßt sich das Verfahren durch folgendes Schema beschreiben:

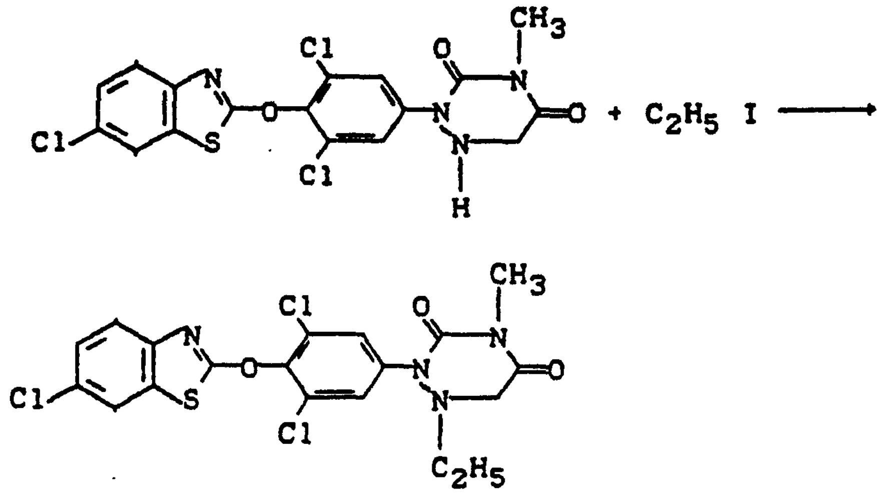

Die Verbindungen der Formel V werden von Formel I umfaßt. Sie sind neu und können wie bei Verfahren 2a) und 2b) beschrieben hergestellt werden. Bevorzugt werden Verbindungen der Formel V eingesetzt, in denen X, $R^1$, $R^2$, $R^3$ die bei den Verbindungen der Formel I angegebenen bevorzugten Bedeutungen besitzen.

Das Verfahren 2c) wird wie unter 2b) beschrieben durchgeführt.

Die neuen Verbindungen der Formel II können nach den unter (4) angegebenen Verfahren hergestellt werden.

Einige Verbindungen der Formel II, in der X für O, S, SO, $SO_2$ steht, sind Gegenstand einer älteren, nicht vorveröffentlichten Anmeldung der Anmelderin (Deutsche Patentanmeldung P 38 05 660. ). Zusätzlich zu den dort angegebenen Herstellverfahren lassen sich die Verbindungen der Formel II, in welcher X für O, S, SO, $SO_2$ steht, nach den im folgenden beschriebenen Verfahren 4b) - d) herstellen.

Setzt man bei dem Verfahren 4a) zur Herstellung der Verbindungen der Formel II, in welcher $R^3$ nicht für Wasserstoff steht, als Verbindung der Formel VIIa 2-[4-(2′-Pyridiloxy)phenyl]1,2,4-triazin-3,5-(2H,4H)-dion und als Verbindung der Formel IV Methyliodid ein, läßt sich das Verfahren durch folgendes Schema beschreiben:

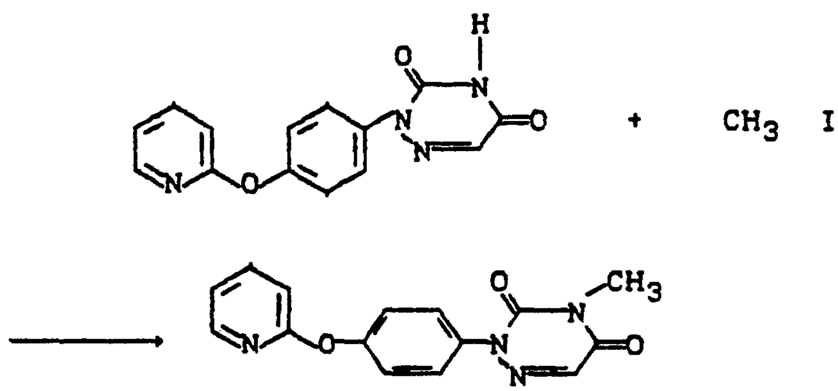

Die Verbindungen der Formel VII werden wie bei Verfahren 4b) beschrieben dargestellt.

Die Verbindungen der Formel IV sind bekannt oder lassen sich nach bekannten Methoden darstellen Besonders genannt sei Methyliodid, Ethylbromid.

Das Verfahren wird durchgeführt, indem man eine Verbindung der Formel VII in Gegenwart einer Base und eines Verdünnungsmittels mit Verbindungen der Formel IV umsetzt. Als Verdünnungsmittel können alle inerten organischen Lösungsmittel eingesetzt werden, die auch zur Durchführung von Verfahren Ia dienen.

Das Verfahren wird in Gegenwart von Basen durchgeführt. Als bevorzugte Basen seien genannt die Alkalihydroxide wie Natriumyhydroxid, Alkalialkoholate wie Natriummethylat oder Kaliumbutanolat, Metallhydride wie Natriumhydrid oder organische Basen wie 1,8-Diazabicyclo[5,40]-undec-7-en (DBU).

Das Verfahren wird durchgeführt bei Normaldruck und Temperaturen zwischen 20° und 140° C.

Die Reaktion wird durchgeführt indem man äquimolare Mengen der Verbindung der Formel VII und Base zusammengibt, dieses Gemisch mit einer äquimolaren Menge der Verbindung der Formel IV versetzt und auf die Reaktionstemperatur erhitzt.

Nach dem im folgenden beschriebenen Verfahren 4b) lassen sich sowohl die Verbindungen der Formel II als auch die Verbindungen der Formel VII herstellen.

Wird bei Verfahren 4b) zur Herstellung der Verbindungen der Formel II als Verbindung der Formel VIII 2-(3-Methyl-4-pyridylphenyl)-1,2,4-triazin-2,5(2H,4H)dion-6-carbonsäure eingesetzt, läßt sich das Verfahren durch folgendes Schema beschreiben:

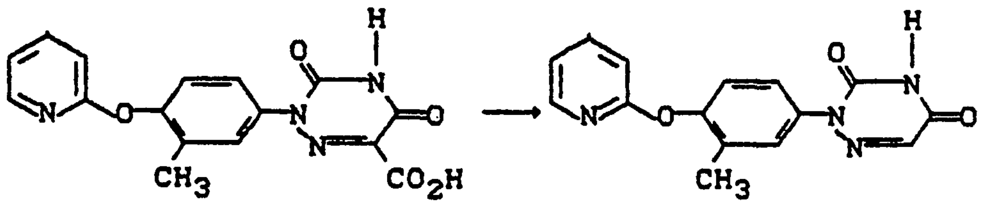

Die Verbindungen der Formel VIII werden nach dem weiter unten (6) beschriebenen Verfahren hergestellt. Es werden bevorzugt Verbindungen der Formel VIII eingesetzt, in denen X, $R^1$, $R^2$, $R^3$ die bei den Verbindungen der Formel I angegebenen bevorzugten Bedeutungen haben.

Als Einzelverbindungen der Formel VIII seien genannt:

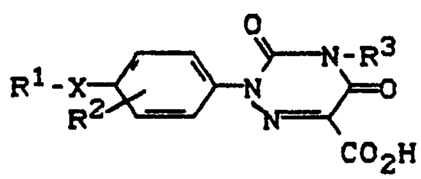

| $R^1$ | X | $R^2$ | $R^3$ |
|---|---|---|---|
| 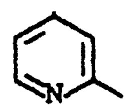 | CH(CN) | 3,5-$Cl_2$ | H |
| " | O | 3,5-$Cl_2$ | H |
| " | O | 3-$CH_3$ | H |
| 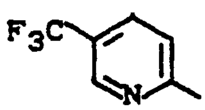 | O | 3,5-$Cl_2$ | H |
| 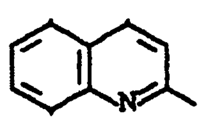 | O | 3,5-$Cl_2$ | H |

| $R^1$ | X | $R^2$ | $R^3$ |
|---|---|---|---|
| 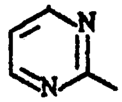 | O | 3,5-$Cl_2$ | H |
| 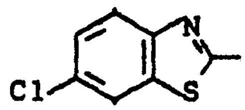 | O | 3,5-$Cl_2$ | H |
| " | O | 3-$CH_3$ | H |
| 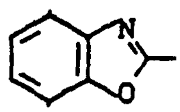 | O | 3,5-$Cl_2$ | H |
| 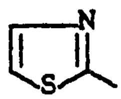 | O | 3,5-Cl | H |
| 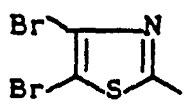 | O | 3,5-$Cl_2$ | H |

Die Decarboxylierung wird gegebenenfalls in Gegenwart von inerten organischen Verdünnungsmitteln durchgeführt. Hierzu gehören aliphatische und aromatische, gegebenenfalls halogenierte Kohlenwasserstoffe wie Nonan, Decan, Dodecan, Xylole, Alkohole wie Diethylenglykol, Ether wie Ethylenglykolmonobutylether, Diethylenglykoldibutylether, Sulfoxide wie Dimethylsulfoxid und Sulfone wie Tetramethylensulfon.

Darüber hinaus kann die Reaktion in Gegenwart von mercaptogruppenhaltigen Carbonsäuren wie z.B. Mercaptoessigsäure oder Thiosalicylsäure durchgeführt werden.

Die Umsetzung erfolgt bei Temperturen zwischen 150 und 300° C, gegebenenfalls in Gegenwart von mercaptogruppenhaltigen Carbonsäuren wie z.B. Mercaptoessigsäure vorzugsweise zwischen 160 und 250° C, insbesondere zwischen 180 und 210C°.

Es wird bei Normaldruck gearbeitet. Die Verbindungen der Formeln VIII werden in Substanz oder im jeweiligen Verdünnungsmittel, gelöst oder suspendiert, erhitzt.

Setzt man im Verfahren 4c) zur Herstellung der Verbindungen der Formel II als Verbindung der Formel IX 1-[3,5-Dichlor-4-(2-pyridyl)-phenyl]-3,3-dimethyl-1,2,4-triazolidin-5-on ein, läßt sich das Verfahren durch folgendes Schema beschreiben:

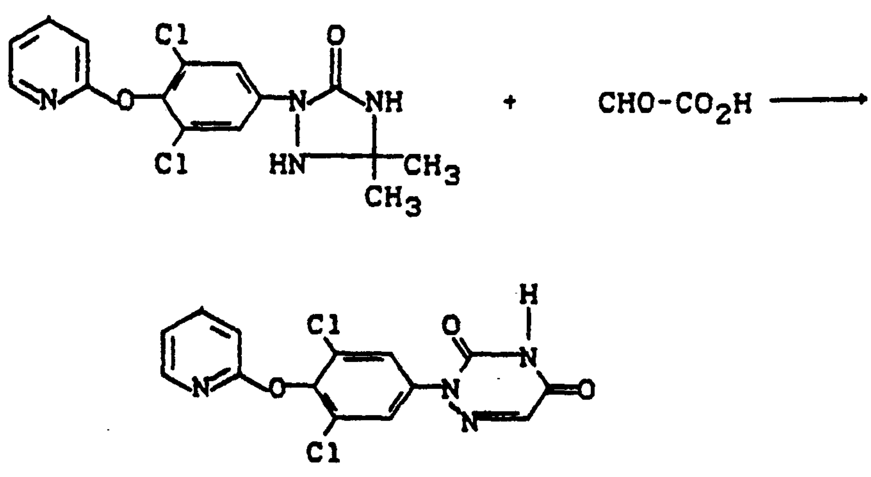


Die Verbindungen der Formel IX sind neu. Ihre Herstellung erfolgt nach dem unter (12) beschriebenen

Verfahren.

Bevorzugt werden Verbindungen der Formel IX eingesetzt, in der X, $R^1$, $R^2$ die bei den Verbindungen der Formel I angegebenen bevorzugten Bedeutungen haben und $R^6$ und $R^7$ unabhängig voneinander für $C_{1-4}$-Alkyl, insbesondere Methyl oder Ethyl stehen. Im einzelnen seien genannt:

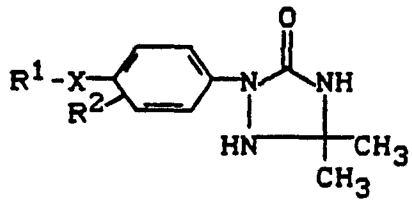

| $R^1$ | X | $R^2$ |
|---|---|---|
| 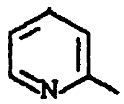 | O | 3,5-$Cl_2$ |
| 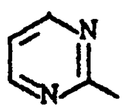 | O | 3,5-$Cl_2$ |
| 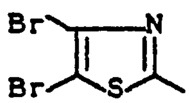 | O | 3,5-$Cl_2$ |
| 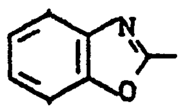 | O | 3,5-$Cl_2$ |
| 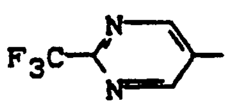 | O | 3,5-$Cl_2$ |

Das Verfahren wird durchgeführt, indem man eine Verbindung der Formel IX in einem Verdünnungsmittel in Gegenwart von Glyoxylsäure und einer katalytischen Menge konzentrierter Mineralsäure erwärmt und das Rohprodukt nach wäßriger Aufarbeitung an Kieselgel chromatographiert.

Als Verdünnungsmittel können alle inerten organischen Lösungsmittel verwendet werden, die z.B. bei Verfahren 2b) angeführt sind. Als Mineralsäure wird vorzugsweise Schwefelsäure verwendet. Es wird bei Temperaturen zwischen 60° C und 130° C, vorzugsweise bei 100° C, gearbeitet.

Wird im Verfahren 4d) zur Herstellung der Verbindungen der Formel II als Verbindung der Formel XI Ethyl-N-[[3-methyl-4-(5′-trifluoromethyl-2′-pyridinyloxyphenyl] hydrazinyliden-carbonyl]carbamat eingesetzt, läßtsich das Verfahren durch folgendes Schema beschreiben:

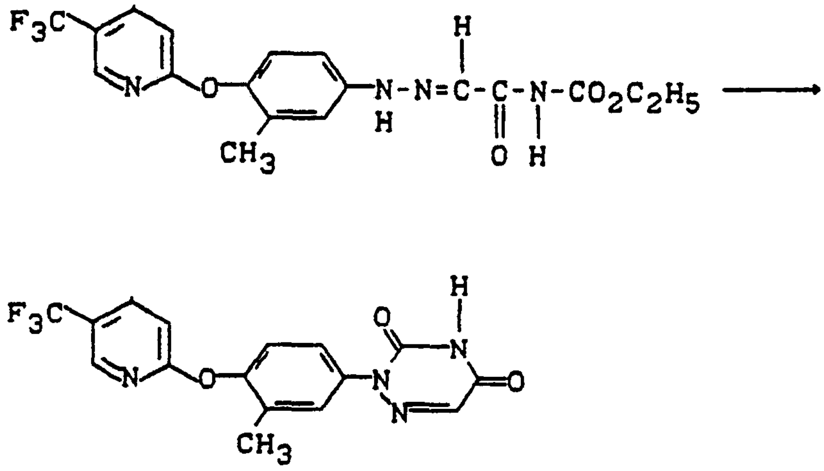

Die Verbindungen der Formel XI sind neu. Ihre Herstellung erfolgt nach dem unter (10) beschriebenen Verfahren. Bevorzugt werden Verbindungen der Formel XI eingesetzt, in der X, $R^1$, $R^2$, $R^3$ die bei den Verbindungen der Formel I angegebenen bevorzugten Bedeutungen haben, $R^8$ für $C_{1-4}$-Alkyl, insbesondere Methyl oder Ethyl und Phenyl steht und $R^9$ für Wasserstoff oder CN steht.

Als Einzelverbindungen dar Formel XI seien genannt:

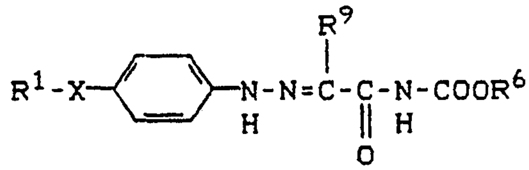

| $R^1$ | X | $R^2$ | $R^4$ | $R^6$ |
|---|---|---|---|---|
| 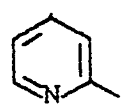 | CHCN | 3,5-$Cl_2$ | H | Et |
| | O | 3,5-$Cl_2$ | H | Et |
| | O | 3-$CH_3$ | H | Et |
| 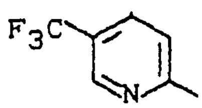 | O | 3,5-$Cl_2$ | H | Et |

Das Verfahren wird durchgeführt, indem man eine Verbindung der Formel XI erhitzt, gegebenenfalls in Gegenwart eines Lösungsmittels und einer Base.

Als Lösungsmittel und Basen finden die bei der Herstellung der Verbindungen I aufgeführten Lösungsmittel und Basen Verwendung. Als weitere besonders bevorzugte organische Lösungsmittel werden Alkohole wie z.B. Ethanol oder organische Säuren wie z.B. Eisessig eingesetzt.

Besonders bevorzugte Basen sind die Hydroxide und Acetate der Alkali- oder Erdalkalimetalle wie z.B. NaOH oder Natrium- und Kaliumacetat.

Die Umsetzung erfolgt unter Normaldruck bei Temperaturen zwischen 70 und 150 °C, vorzugsweise zwischen 70 und 100 °C.

Die verwendete Base wird in 10-80 %igem molarem Überschuß eingesetzt. Das Reaktionsgemisch wird nach abgeschlossener Cyclisierung vorzugsweise mit einer verdünnten Mineralsäure wie z.B. Salzsäure angesäuert und das als Feststoff anfallende Produkt abfiltriert.

Die bei Verfahren 4b) eingesetzten Verbindungen der Formel VIII sind neu. Sie werden nach dem unter (6) beschriebenen Verfahren hergestellt.

Setzt man bei dem Verfahren 4e) als Verbindung XVII 2-(3,5-Dichloro-4-hydroxyphenyl)-1,2,4-triazin-3,5-(2H,4H)-dion und als Verbindung der Formel III 2-Chlorpyridin ein und läßt sich das Verfahren durch folgendes Formelschema beschreiben.

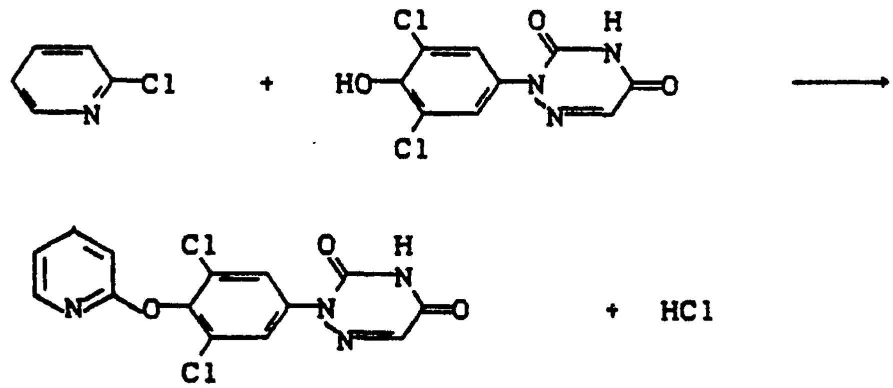

Verbindungen der Formel XVII
in welcher
$R^2$ und $R^3$ für Wasserstoff stehen, sind bekannt (J. Slouka, Acta Unio Palacki Olomuk. Fac. Rerum. Nat. 1984 (Chem 23), 39 - 45; C.A. 102 203946c).

Andere Verbindungen der Formel XVII sind Gegenstand einer bislang nicht veröffentlichen Ameldung der Anmelderin (Deutsche Patentanmeldung P 3 805 660). Sie lassen sich analog zu den für die Verbindungen der Formel II angegebenen Verfahren herstellen.

Bevorzugt seien Verbindungen der Formel XVII genannt, in denen $R^2$ und $R^3$ die bei den Verbindungen der Formel I genannten bevorzugten Bedeutungen haben.

Die substituierten Heterocyclen der Formel XVIII sind bekannt oder lassen sich analog zu bekannten Verfahren herstellen (Beilstein Bd. 27; Katrizky und Rees, Comprehensive Het. Chem. Col. 6 1984).

Sie besitzen die weiter oben bei den Verbindungen der Formel I angegebenen bevorzugten Bedeutungen. Im einzelnen seien die folgenden Verbindungen der Formel XVIII genannt:

XVIII

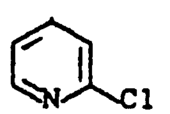 ,

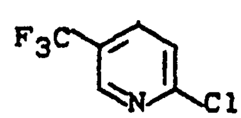

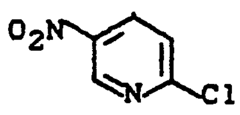 ,

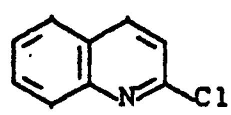

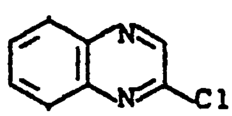 ,

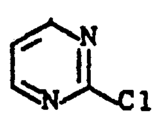

Die Reaktion wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuertriamid.

Die Reaktion wird in Gegenwart von anorganischen oder organischen Säureakzeptoren durchgeführt.

Als solche seien z.B. genannt:

Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kaliummethylat bzw. -ethylat, ferner aliphatische, aromatische oder heterocyclische Amine, beispielsweise Triethylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Umsetzung erfolgt bei Temperaturen zwischen 50 und 200° C, vorzugsweise zwischen 80 und 160° C bei Normaldruck oder erhöhtem Druck. Es wird bevorzugt bei Normaldruck gearbeitet.

Das Verfahren wird durchgeführt, indem äquimolare Mengen der Verbindungen der Formel XVII und XVIII in einem der angegebenen Verdünnungsmittel zusammengegeben und erhitzt werden. Nach vollendeter Umsetzung wird das Reaktionsgemisch mit verdünnter anorganischer Säure (z.B. Salzsäure) angesäuert und der entstehende Niederschlag abfiltriert, gewaschen und getrocknet.

Wird im Verfahren 6 zur Herstellung der Verbindungen der Formel VIII als Verbindung der Formel XII 2-[3′-Methyl-4′-(2-trifluoromethyl-3-pyridinyl)phenyl]-6-cyano-1,2,4-triazin-3,5(2H,4H)dion eingesetzt, läßt sich das Verfahren durch folgendes Formelschema beschreiben:

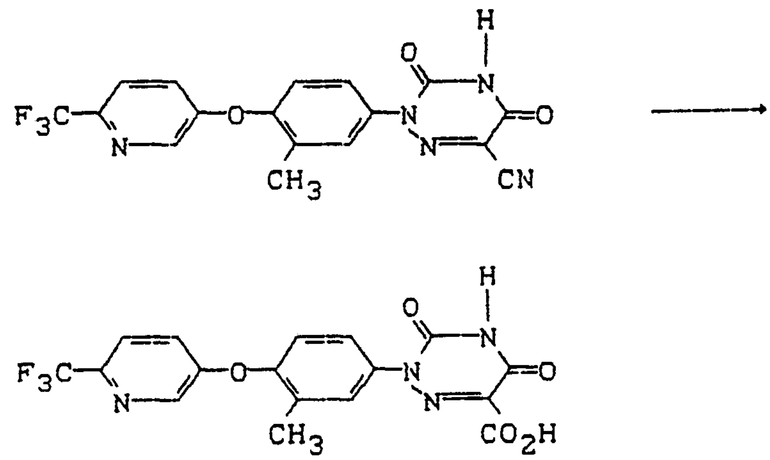

Die Verbindungen der Formel XII sind neu. Ihre Herstellung erfolgt nach dem unter (8) beschriebenen Verfahren. Bevorzugt werden Verbindungen der Formel XII eingesetzt, in der X, $R^1$, $R^2$, $R^3$ die bei den Verbindungen der Formel I angegebenen bevorzugten Bedeutungen hat und $R^9$ für CN steht.

Als Einzelverbindungen der Formel XII seien genannt:

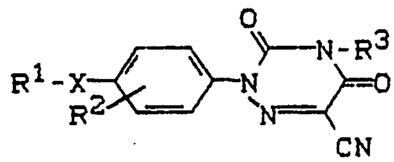

| $R^1$ | X | $R^2$ | $R^3$ |
|---|---|---|---|
| 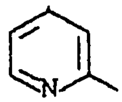 | CHCN | 3,5-$Cl_2$ | H |
| " | O | 3,5-$Cl_2$ | H |
| " | O | 3-$CH_3$ | H |
| 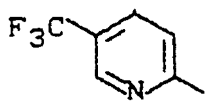 | O | 3,5-$Cl_2$ | H |
| 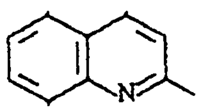 | O | 3,5-$Cl_2$ | H |
| " | O | 3-$CH_3$ | |
| 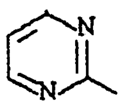 | O | 3,5-$Cl_2$ | H |
| 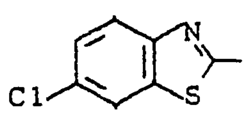 | O | 3,5-$Cl_2$ | H |
| " | O | 3-$CH_3$ | H |
| 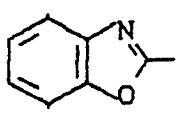 | O | 3,5-$Cl_2$ | H |
| 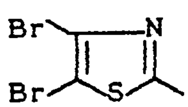 | O | 3,5-$Cl_2$ | H |
| 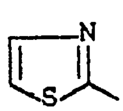 | O | 3,5-$Cl_2$ | H |

Die Hydrolyse der Verbindungen der Formel XII wird unter sauren Bedingungen durchgeführt. Als Säuren kommen Mineralsäuren zum Einsatz wie z.B. Salzsäure, Bromwasserstoffsäure, Schwefelsäure und Gemische aus Mineralsäuren und organischen Säuren wie z.B. Essigsäure oder Propionsäure.

Die Umsetzung erfolgt bei Temperaturen zwischen 80 und 120 °C. Es wird unter Normaldruck gearbeitet.

Die Verbindungen der Formel XII werden im 10-30-fachen Volumen der Säure oder des Säuregemisches gelöst und bis zur abgeschlossenen Hydrolyse erhitzt.

Das Verfahren kann auch so geführt werden, daß ohne Isolierung der Verbindung der Formel VIII direkt Verbindungen der Formel II entstehen. Dazu wird die Hydrolyse der Verbindung der Formel XII unter Zusatz von mercaptogruppenhaltigen Carbonsäuren wie z.B. Mercaptoessigsäure oder Thiosalicylsäure durchgeführt.

Wird im Verfahren 8 zur Herstellung der Verbindungen der Formel XII als Verbindung der Formel XI Ethyl-N-[[[Cyano(3-methyl-4-pyridyloxyphenyl)-hydrazinyliden]methyl]carbonyl]-carbamat eingesetzt, läßt sich das Verfahren durch folgendes Schema beschreiben:

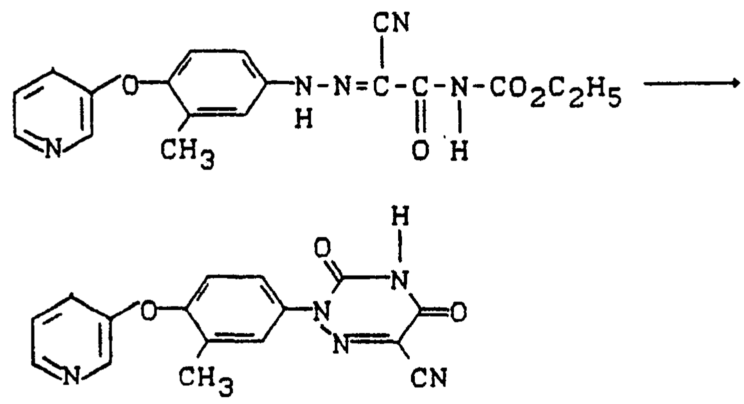

Die Verbindungen der Formel XI sind neu. Ihre Herstellung erfolgt nach dem unter (10) beschriebenen Verfahren. Bevorzugt werden Verbindungen der Formel XI eingesetzt, in der X, $R^1$, $R^2$, $R^3$ die bei den Verbindungen der Formel I angegebenen bevorzugten Bedeutungen haben, $R^8$ für $C_{1-4}$-Alkyl, insbesondere Methyl oder Ethyl und Phenyl steht, und $R^9$ für CN steht.

Als Einzelverbindungen der Formel XI seien genannt:

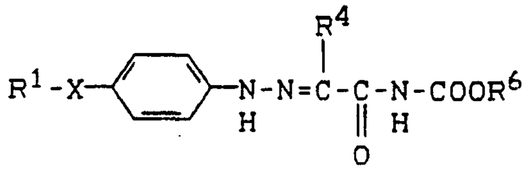

| $R^1$ | X | $R^2$ | $R^4$ | $R^6$ |
|---|---|---|---|---|
| 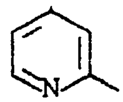 | CHCN | 3,5-$Cl_2$ | CN | Et |
| " | O | 3,5-$Cl_2$ | CN | Et |
| " | O | 3-$CH_3$ | CN | Et |
| 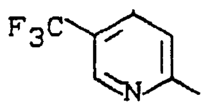 | O | 3,5-$Cl_2$ | CN | Et |
| 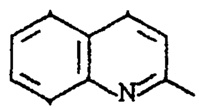 | O | 3,5-$Cl_2$ | CN | Et |
| " | O | 3-$CH_3$ | CN | Et |
| 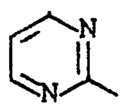 | O | 3,5-$Cl_2$ | CN | Et |
| 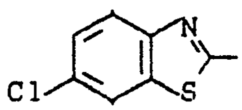 | O | 3,5-$Cl_2$ | CN | Et |
| " | O | 3-$CH_3$ | CN | Et |

| $R^1$ | X | $R^2$ | $R^4$ | $R^6$ |
|---|---|---|---|---|
| 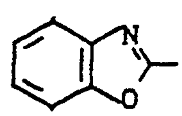 | O | 3,5-$Cl_2$ | CN | Et |
| 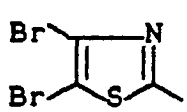 | O | 3,5-$Cl_2$ | CN | Et |
| 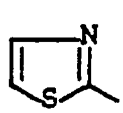 | O | 3,5-$Cl_2$ | CN | Et |

Das Verfahren (8) wird wie bei Verfahren 4d) beschrieben durchgeführt.

Die in den Verfahren 4d) und 8 eingesetzten Verbindungen der Formel XI werden nach dem unter 10 beschriebenen Verfahren hergestellt.

Wird im Verfahren 10a) zur Herstellung der Verbindungen der Formel XI als Verbindung der Formel XIII 3-Methyl-4-benzthiazolyloxyanilin eingesetzt und als Verbindung der Formel XIV Ethyl-cyanacetylurethan läßt sich das Verfahren durch folgendes Schema beschreiben:

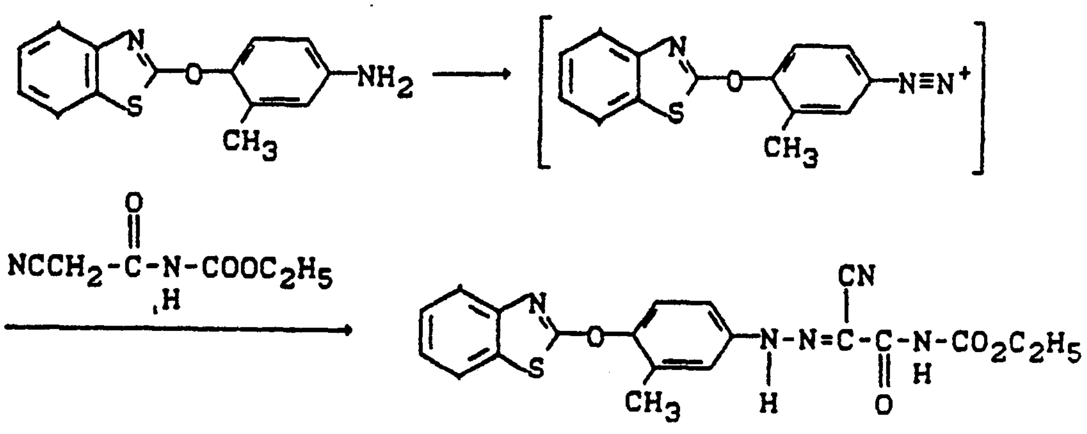

Die Verbindungen der Formeln XIII und XIV sind bekannt oder lassen sich analog zu bekannten Verfahren herstellen (vgl. DE-OS 34 05 241).

Das Verfahren wird durchgeführt, indem man ein Anilin der Formel XIII mit $NaNO_2$ und konz Mineralsäure wie z.B. HCl, gegebenenfalls in Gegenwart eines Verdünnungsmittels, umsetzt.

Als Verdünnungsmittel dienen mit Wasser mischbare Verdünnungsmittel wie Alkohole, z.B. Methanol, Ethanol, organische Säuren wie z.B. Eisessig, Ameisensäure, Glycolether wie Monomethylglycolether, Nitrile wie Acetonitril, Dimethylsulfoxid.

Das so erzeugte Diazoniumsalz wird in situ mit einer Verbindung der Formel XIV wie z.B. Malonyldiurethan oder Cyanacetylurethan in Gegenwart einer Base umgesetzt. Als Basen finden Verwendung Hydroxide und Carbonate der Alkali und Erdalkalimetalle sowie Acetate von Natrium, Kalium und Ammonium.

Weiterhin können organische Basen wie Pyridin oder Triethylamin verwendet werden.

Die Diazotierung wird bei Normaldruck und bei Temperaturen zwischen 0° C und 10° C durchgeführt. Die Zugabe der Verbindungen der Formel XIV erfolgt bei 5° bis 20° C. Anilin und Nitrit werden in äquimolaren Mengen in einem Überschuß Säure vorzugsweise der 2-3 fachen molaren Menge umgesetzt. Die CH-acide Verbindung wird in 0 bis 30 %igem molaren Überschuß, vorzugsweise 10 %igem Überschuß zugesetzt. Die Base wird im 1,5-2,5-fachen molaren Überschuß zugesetzt.

Das Kupplungsprodukt aus Diazoniumsalz und CH-acider Verbindung ist im Reaktionsmedium unlöslich und kann als Feststoff isoliert werden.

Das Verfahren kann auch so geführt werden, daß ohne Isolierung der Verbindung der Formel XI direkt Verbindungen der Formel VIII entstehen. Dazu wird die Diazotierung der Aniline der Formel XIII und die Umsetzung mit den Urethanen der Formel XIV in einem für die Cyclisierung geeigneten Verdünnungsmittel durchgeführt. Das Reaktionsgemisch wird nach erfolgter Diazotierung und Kupplung erwärmt und dann das Triazindion der Formel VIII isoliert.

Als Verdünnungsmittel seien genannt: Alkohole wie Methanol, Ethanol.

Zur Cyclisierung wird das Reaktionsgemisch auf ca. 80 bis 120° C, bevorzugt ca. 80 bis 100° C, erwärmt.

Die Aufarbeitung erfolgt wie weiter oben bei Verfahren (6) zur Herstellung der Verbindungen der Formel VIII angegeben.

Wird im Verfahren 10b) zur Herstellung der Verbindungen XI als Verbindung der Formel XV 3,5-Dichloro-4-(2-pyridyl)-phenylhydrazin eingesetzt, läßt sich das Verfahren durch folgendes Schema beschreiben:

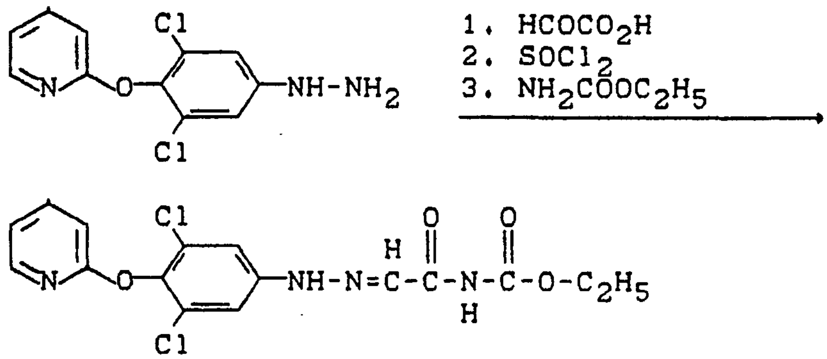


Die Verbindungen der Formeln XV und XIV sind bekannt oder lassen sich analog zu bekannten Verfahren herstellen. Es werden bevorzugt Verbindungen eingesetzt, in denen X, $R^1$, $R^2$, $R^3$ die bei den Verbindungen der Formel I angegebenen bevorzugten Bedeutungen besitzen, $R^9$ für Wasserstoff oder CN steht und $R^8$ für $C_{1-4}$-Alkyl, insbesondere Methyl oder Ethyl steht.

Das Verfahren wird durchgeführt, indem man eine Verbindung der Formel XV zunächst mit Glyoxylsäure umsetzt. Das Rohprodukt der Reaktion wird mit Thionylchlorid in das jeweilige Säurechlorid umgewandelt und dieses dann mit Ethylurethan umgesetzt. Die erste Stufe der Reaktion wird in Gemische aus Alkoholen, vorzugsweise Ethanol und Wasser, durchgeführt. Die zweite und dritte Stufe des Verfahrens können in Gegenwart der bei Verfahren 2a) beschriebenen inerten organischen Lösungsmittel durchgeführt werden. Bevorzugt seien für die zweite Stufe Kohlenwasserstoffe wie Toluol genannt. Die Reaktionstemperatur kann zwischen 20 und 120 °C variiert werden, wobei die 1. Stufe vorzugsweise bei 20 bis 40 °C, die 2. und 3. Stufe bei 80 bis 110 °C durchgeführt werden.

Die Verbindungen der Formel IX lassen sich nach Verfahren 12 herstellen.

Wird im Verfahren 12 zur Herstellung der Verbindungen IX als Verbindung der Formel XV 3,5-Dichloro-4-(2-pyridyloxy)-phenylhydrazin eingesetzt, läßt sich das Verfahren durch folgendes Schema beschreiben:

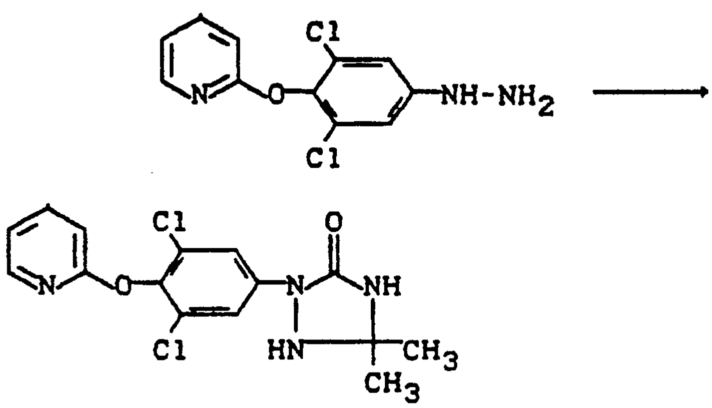

Es werden Verbindungen der Formel XV eingesetzt, in denen X, $R^1$, $R^2$ die bei den Verbindungen der Formel I angegebenen bevorzugten Bedeutungen besitzen. Es werden Ketone der Formel XVI eingesetzt, in denen $R^6$ und $R^7$ für $C_{1-4}$-Alkyl, insbesondere Methyl oder Ethyl stehen.

Das Verfahren wird durchgeführt, indem man eine Verbindung der Formel XV mit einem Keton wie z.B. Aceton umsetzt. Das so hergestellte Hydrazon wird mit Alkalicyanat, insbesondere Kaliumcyanat, umgesetzt. Die Reaktion kann in Gegenwart von inerten organischen Lösungsmitteln durchgeführt werden, wie sie auch in Verfahren 10b) Verwendung finden. Die beiden Stufen werden bei Temperaturen zwischen 20 und 40 °C durchgeführt.

Die Wirkstoffe eignen sich bei günstiger Warmblütertoxizität zur Bekämpfung von parasitischen Protozoen die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Stämme wirksam. Durch die Bekämpfung der parasitischen Protozoen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und

einfachere Tierhaltung möglich ist.

Zu den parasitischen Protozoen zählen:

Mastigophora (Flagellata) wie z.B. Trypanosomatidae z.B. Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthamoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. dabliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I.rivolta, I. spec., I. suis, Cystisospora spec., Cryptosporidium spec. wie Toxoplasmadidae z.B. Toxoplasma gondii, wie Sarcocystidae z.B. Sarcocystis bovicanis, S. bovihominis, S. ovicanis, S. ovifelis, S. spec., S. suihominis wie Leucozoidae z.B. Leucozytozoon simondi, wie Plasmodiidae z.B. Plasmodium berghei, P. falciparum, P. malariae, P. ovale, P. vivax, P. spec., wie Piroplasmea z.B. Babesia argentina, B. bovis, B. canis, B. spec., Theileria parva, Theileria spec., wie Adeleina z.B. Hepatozoon canis, H. spec.

Ferner Myxospora und Microspora z.B. Glugea spec. Nosema spec.

Ferner Pneumocystis carinii, sowie Ciliophora (Ciliata) wie z.B. Balantidium coli, Ichthiophthirius spec., Trichodina spec., Epistylis spec.

Die erfindungsgemäßen Verbindungen sind auch wirksam gegen Protozoen, die als Parasiten bei Insekten auftreten. Als solche seien genannt Parasiten des Stammes Microsporida, insbesondere der Gattung Nosema. Besonders genannt sei Nosema apis bei der Honigbiene.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z. B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z. B. Plagioscion, Channel catfish. Besonders geeignet sind die erfindungsgemäßen Mittel zur Behandlung von Fischbrut, z. B. Karpfen von 2 - 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzen trate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind Z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren,

Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester Ethyloleat, Milchsäureethylester, wachsartige Fettsäureester wie Dibutylphthalat Adipinsäurediisopropylester letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt: Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside wie Na-Laurylsulfat Fettalkoholethersulfate, Mono/Dialkylpolyglykoletherorthophosphorsäureester-monoethanolaminsalz;
kationaktive Tenside wie Cetyltrlmethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffen Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische

Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Täger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten Die hierbei erhaltene Vormischung kann dann dem vollständigen Futtermittel vor seiner Verfütterung an die Tiere zugesetzt werden.

Beispielhaft sei der Einsatz bei der Coccidiose genannt:

Für die Heilung und Prophylaxe etwa der Coccidiose bei Geflügel, insbesondere bei Hühnern, Enten, Gänsen und Truthähnen, werden 0,1 bis 100 ppm, vorzugsweise 0,5 bis 100 ppm eines Wirkstoffs mit einem geeigneten eßbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Für die Behandlung von Einzeltieren, z.B. im Falle der Behandlung der Coccidiose bei Säugetieren oder der Toxoplasmose, werden vorzugsweise Wirkstoffmengen von 0,5 bis 100 mg/kg Körpergewicht täglich verabreicht, um die gewünschten Ergebnisse zu erzielen. Trotzdem kann es zeitweilig notwendig sein, von den genannten Mengen abzuweichen, insbesodnere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muß. Bei der Verabreichung größerer Mengen kann es zweckmäßig sein, diese im Verlauf des Tages in mehrere Einzeldarreichungen zu unterteilen.

Darüber hinaus sind die erfindungsgemäßen Verbindungen wirksam gegenüber verschiedenen zu den Helminthen (Würmern) zählenden Fischparasiten.

Zu den Parasiten bei Fischen gehören aus dem Unterreich der Protozoen Spezies des Stammes der Ciliata, z.B. Ichthyophthirius multifiliis, Chilodonella cyprini, Trichodina spp., Glossatella spp., Epistylis spp. des Stammes der Myxosporidia, z.B. Myxosoma cerebralis, Myxidium spp., Myxobolus spp., Heneguya spp., Hoferellus spp., der Klasse der Mikrosporidia z.B. Glugea spp., Thelohania spp., Pleistophora spp., aus dem Stamm der Plathelminthen: Trematoden; Monogenea z.B. Dactylogyrus spp., Gyrodactylus spp., Pseudodactylogyrus spp., Diplozoon spp., Cestoden, z.B. aus den Gruppen der Caryphyllidea (z.B. Caryophyllaeus laticeps), Pseudophyllidea (z.B. Diphyllobothrium spp.), Tetraphyllidea (z.B. Phyllobothrium spp.) und Protocephalida (z.B. Arten der Gattung Proteocephalus) und aus dem Stamm der Arthropoda verschiedene parasitische Crustaceen, insbesondere aus den Unterklassen der Branchiura (Fischläuse) und Copepoda (Ruderfußkrebse) sowie den Ordnungen der Isopoda (Asseln) und Amphipoda (Flohkrebse).

Die Behandlung der Fische erfolgt entweder oral, z. B. über das Futter oder durch Kurzzeitbehandlung, "medizinisches Bad", in das die Fische eingesetzt und in dem sie eine Zeitlang (Minuten bis mehrere Stunden) z. B. beim Umsetzen von einem Zuchtbecken zum anderen gehalten werden.

Es kann aber auch eine vorübergehende oder dauernde Behandlung des Lebensraums der Fische (z. B. ganzer Teichanlagen, Aquarien, Tanks oder Becken), in denen die Fische gehalten werden, erfolgen.

Der Wirkstoff wird in Zubereitungen verabreicht, die den Anwendungen angepaßt sind.

Die Konzentration des Wirkstoffs, liegt in den Zubereitungen bei 1 ppm bis 10 Gew.-%.

Bevorzugte Zubereitungen zur Kurzzeitbehandlung in der Anwendung als "medizinisches Bad" z.B. bei der Behandlung beim Umsetzen der Fische oder zur Behandlung des Lebensraums (Teichbehandlung) der Fische sind Lösungen des Wirkstoffs in einem oder mehreren polaren Lösungsmitteln, die bei Verdünnen mit Wasser alkalisch reagieren.

Zur Herstellung dieser Lösungen wird der Wirkstoff in einem polaren, wasserlöslichen Lösungsmittel gelöst, welches entweder alkalisch reagiert oder dem eine alkalische wasserlösliche Substanz zugefügt wird. Letztere wird vorteilhaft ebenfalls im Lösungsmittel gelöst, kann aber auch in dem Lösungsmittel suspendiert sein und sich erst im Wasser lösen. Dabei soll das Wasser nach Zusatz der Wirkstofflösung einen pH-Wert von 7-10, vorzugsweise aber einen pH-Wert von 8-10 haben.

Die Konzentration des Wirkstoffes kann im Bereich von 0,5-50 % liegen, vorzugsweise aber in einem Bereich von 1-25 %.

Als Lösungsmittel kommen alle wasserlöslichen Lösungsmittel in Betracht, in denen der Wirkstoff in genügender Konzentration löslich ist und die physiologisch unbedenklich sind.

Dies sind Ethylalkohol, Isopropylalkohol, Benzylalkohol, Glycerin, Propylenglykol, Polyethylenglykole, Poly(oxoethylen)-poly(oxypropylen)-Polymere, basische Alkohole wie Mono-, Di- und Triethanolamin, Ketone wie Aceton oder Methylethylketon, Ester wie Milchsäureethylester ferner N-Methylpyrrolidon, Dimethylacetamid, Dimethylformamid, ferner Dispergier- und Emulgiermittel wie polyoxyethyliertes Rizinusöl, Polyethylenglykol-Sorbitan-Monooleat, Polyethylenglykolstearat, oder Polyethylenglykolether, Polyethylenglykol-Alkylamine.

Als Basen zur Einstellung des alkalischen pH-Wertes seien genannt organische Basen wie basische Aminosäuren wie L- bzw. D,L-Arginin, L- bzw. D, L-Lysin, Methylglucosamin, Glucosamin, 2-Amino-2-hydroxymethylpropandiol-(1,3) ferner wie N,N,N′,N′-tetrakis-(2-hydroxypropyl)-ethylendiamin oder Polyether-Tetrol auf der Basis Ethylendiamin (M.G. 480-420), anorganische Basen, wie Ammoniak oder Natriumcarbonat-gegebenenfalls unter Zugabe von Wasser.

Die Zubereitungen können auch 0,1 bis 20 Gew-%, vorzugsweise 0,1-10 Gew-% anderer Formulierhilfsstoffe, wie Antioxydantien, Tenside, Suspensionsstabilisatoren und Verdickungsmittel wie z.B. Methylcellulose, Alginate, Polysaccharide, Galaktomannane und kolloidale Kieselssäure enthalten. Der Zusatz von Farbe, Aroma und Aufbaustoffen zur Tierernährung ist ebenfalls möglich. Auch Säuren, die mit der vorgelegten Base zusammen ein Puffersystem bilden oder den pH der Lösung reduzieren, sind hier zu nennen.

Die Konzentration des Wirkstoffs bei der Anwendung hängt ab von Art und Dauer der Behandlung, sowie Alter und Zustand der behandelten Fische. Sie beträgt z.B. bei Kurzzeitbehandlung 2-50 mg Wirkstoff pro Liter Wasser bevorzugt 5-10 mg pro Liter, bei einer Behandlungsdauer von 3-4 Stunden. Bei der Behandlung von jungen Karpfen wird z.B. mit einer Konzentration von 5-10 mg/l und einer Behandlungsdauer von ca. 1-4 Stunden gearbeitet.

Aale werden mit Konzentrationen von ca. 5 mg/l ca. 4 Stunden behandelt.

Bei längerer Behandlungsdauer oder bei Dauerbehandlung kann die Konzentration entsprechend niedriger gewählt werden.

Bei Teichbehandlungen können 0,1-5 mg Wirkstoff pro Liter Wasser verwendet werden.

Zubereitungen zur Anwendung als Futterzusatz sind z.B. wie folgt zusammengesetzt:

| | |
|---|---|
| a) Wirkstoff der Formel I | 1 - 10 Gewichtsteile |
| Sojabohnen-Protein | 49 - 90 Gewichtsteile |

| | |
|---|---|
| b) Wirkstoff der Formel I | 0,5 - 10 Gewichtsteile |
| Benzylalkohol | 0,08 - 1,4 Gewichtsteile |
| Hydroxypropylmethylcellulose | 0 - 3,5 Gewichtsteile |
| Wasser | Rest ad 100 |

Zubereitungen zur Anwendung bei "medizinischen Bädern" und zur Teichbehandlung sind z.B. wie folgt zusammengesetzt und hergestellt.

c) 2,5 g Wirkstoff der Formel (I) werden in 100 ml Triethanolamin unter Erwärmen gelöst.

d) 2,5 g Wirkstoff der Formel (I) 12,5 g Milchsäure werden in 100 ml Triethanol amin unter Erwärmen und Rühren gelöst.

e) 10,0 g Wirkstoff der Formel (I) wird in 100 ml Monoethanolamin gelöst.

| | |
|---|---|
| f) Wirkstoff der Formel I | 5,0 g |
| Propylenglykol | 50,0 g |
| Natriumcarbonat | 5,0 g |
| Wasser | ad 100 ml |

| | |
|---|---|
| g) Wirkstoff der Formel I | 5,0 g |
| Monoethanolamin | 10 g |
| N-Methylpyrrolidon | ad 100 ml |

| | |
|---|---|
| h) Wirkstoff der Formel I | 2,5 g |
| Natriumcarbonat | 5,0 g |
| Polyethylenglykol200 | ad 100 ml |

Der Wirkstoff wird unter Erwärmen im Polyethylenglykol gelöst und Natriumcarbonat darin suspendiert.

Beispiel A

Coccidiose bei Hühnern

9 bis 11 Tage alte Küken wurden mit 40000 sporulierten Oozysten von stark virulenten Stämmen von Eiveria acervulina, E. maxima und E. tenella, den Krankheiteserregern der intestinalen Coccidiose infiziert.

3 Tage vor der Infektion und 8 Tage nach der Infektion (Ende des Versuchs) wurde Wirkstoff in der angegebenen Konzentration im Futter der Tiere eingemischt verabreicht.

Die Zahl der Oozysten im Kot wurde mit Hilfe der Mcmaster-Kammer bestimmt (siehe Engelbrecht und Mitarbeiter "Parasitologische Arbeitsmehoden in Medizin und Veterinärmedizin", S. 172, Akademie-Verlag, Berlin (1965)).

Als wirksam werden diejenigen Dosen angesehen, die die Ausscheidung von Oozysten und/oder klinische Symptome der Coccidiose einschließlich der Mortalität vollständig oder in hohem Maße verhüteten. In der folgenden Tabelle werden die wirksamen Dosen angegeben:

Tabelle 1

| Coccidiose bei Hühnern | | | | | |
|---|---|---|---|---|---|
| Beispiel Nr. | Dosis ppm. | Sterberate tot/eingesetzt | Oocystenausscheidung in % im Vergleich zur unbehandelten infizierten Kontrolle | Gewichtszunahme in % im Vergleich zur nicht infizierten unbehandelten Kontrolle | Blutausscheidung mit dem Kot |
| unbehandelte infizierte Kontrolle | | 2/6 | 100 | 35 | stark |
| 1 | 50 | 0/3 | 0 | 100 | keine |

## Herstellungsbeispiele

I Beispiele für Verfahren 2a)

### Beispiel 1

2-[4-[(6′-Chloro)-2′-benzthiazolyloxy]-3,5-dichlorophenyl]-hexahydro-1,2,4-triazin-3,5-dion

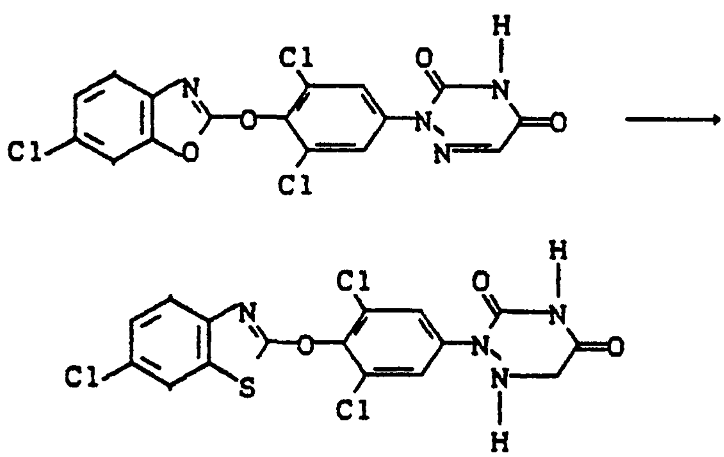

10 g (23 mmol) Benzthiazolyloxyphenylazauracil werden in 100 ml Eisessig gelöst und mit 8 g Zn-Pulver versetzt. Man rührt 2 h unter Rückfluß und filtriert von ungelöstem Feststoff ab. Der Rückstand wird mehrmals mit DMF ausgekocht und abgesaugt. Die vereinigten Filtrate werden zur Trockene eingeengt und der Rückstand aus Ethanol umkristallisiert. Man erhält so 6,5 g (64 % d. Th.) Hexahydroazauracil.

Analog werden hergestellt:

### Beispiel 2

2-[4-[(5,6-Dichloro)-2′-benzthiazolyloxy]-3,5-dichlorophenyl]-hexahydro-1,2,4-triazin-3,5-dion.

### Beispiel 3

2-[4-[(6-Chloro)-2′-benzoxazolyloxy]-3,5-dichlorophenyl]-hexahydro-1,2,4-triazin-3,5-dion

II Beispiele für Verfahren 2b)

### Beispiel 4

2-[4-[(6′-Chloro)-2′-benzthiazolyloxy]-3,5-dichlorophenyl]-hexahydro-1,2,4-triazin-3,5-dion

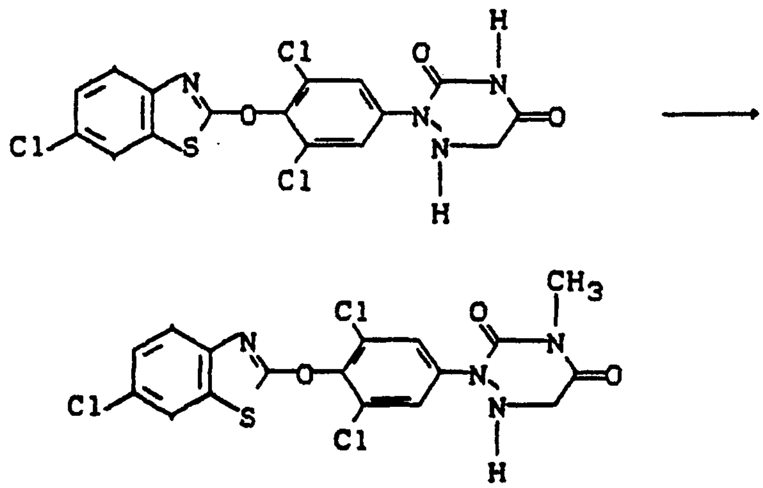

2,5 g (6 mmol) 2-[4-[(6′-Chloro)-2′-benzothiazolyloxy]-3,5-dichlorophenyl]-hexahydro-1,2,4-triazin-3,5-dion werden in 25 ml trockenem DMF gelöst und mit 0,15 g (6 mmol) NaH versetzt. Man rührt 10 min nach und tropft dann 1 g (7 mmol) Methyliodid zu und rührt 5 h bei Raumtemperatur nach. Im Anschluß verdünnt man mit Wasser und saugt den auskristallisierten Feststoff ab. Umkristallisation aus Ethanol liefert 1,9 g (74 % d. Th.) Methylverbindung.

Analog werden hergestellt:

Beispiel 5

2-[4-[(5,6-Dichloro)-2′-benzthiazolyloxy]-3,5-dichlorophenyl]-4-methyl-hexahydro-1,2,4-triazin-3,5-dion.

Beispiel 6

2-[4-[(5,6-Dichloro)-2′-benzthiazolyloxy]-3,5-dichlorophenyl]-4-ethyl-hexahydro-1 ,2,4-triazin-3,5-dion.

III Beispiel für Verfahren 4a)

Beispiel 7

2-[4-(2-Pyridyloxy)phenyl]-3-N-methyl-3-N-methyl-3,5-(2H,4H)-dioxo-1,2,4-triazin

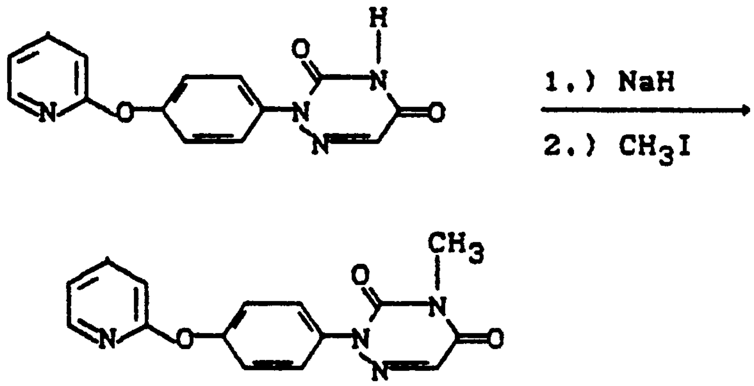


2 g (7 mmol) Pyridinyloxyarylazauracil werden in 20 ml absolutem DMSO gelöst und mit 0,16 g (6 mmol) Natriumhydrid versetzt. Man rührt 20 min bei RT und gibt dann 1,5 g (9 mmol) Methyljodid in 5 ml DMSO unter Argon zu. Man erwärmt auf 50 °C und hält 3 h bei dieser Temperatur. Anschließend wird das Reduktionsgemisch i. V. eingeengt und dann mit Wasser versetzt. Nach dem Absaugen des ausgefallenen Feststoffs erhält man so 1,5 g (72 % der Theorie) der N-Methylverbindung.

IV Beispiel für Verfahren 4b)

Beispiel 8

2-(2-Pyridyloxyphenyl)-1,2,4-triazin-3,5(2,4H)dion

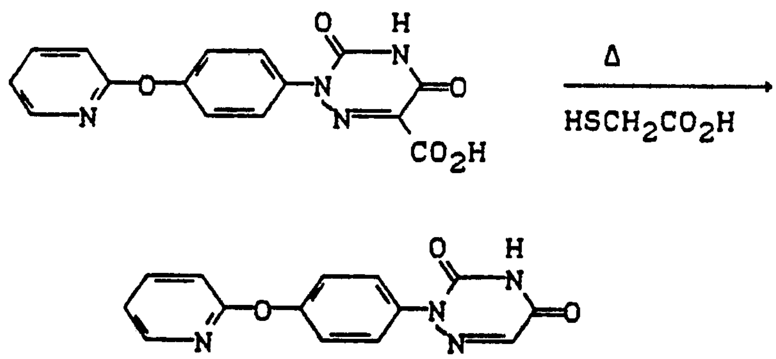


10 g (0,03 mol) Carbonsäure werden in 20 ml Mercaptoessigsäure auf 170 °C erhitzt. Nach 1,5 h läßt man abkühlen, versetzt mit Wasser und erhält nach Abfiltration 27 (82 % der Theorie) decarboxyliertes Produkt.

Beispiel für Verfahren 4c)

Beispiel 9

1-[3,5-Dichloro-4-(2-pyridyl)-phenyl]-1,2,4-triazin-3,5-(2H,4H)-dion

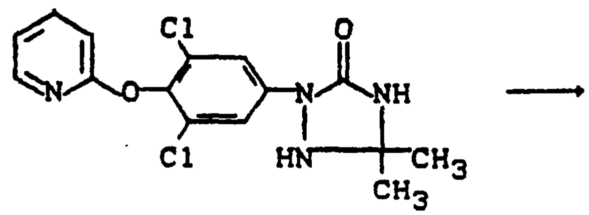

$C_{15}H_{14}Cl_2N_4O_2$

352

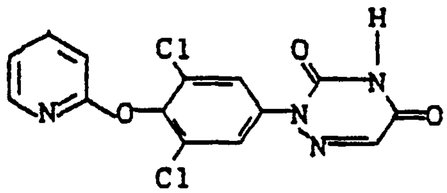

$C_{18}H_8Cl_2N_4O_3$

350

3 g (8,5 mmol) 1-[3,5-Dichloro-4-(2-pyridyl)-phenyl]-3,3-dimethyl-1,2,4-triazolidin-5-on werden in 50 ml Dioxan gelöst und mit 0,78 g (8,5 mmol) Glyoxylsäuremonohydrat und 0,1 ml $H_2SO_4$ konz. versetzt. Man rührt zunächst bei Raumtemperatur 2 h und gibt dann weitere 0,8 g Glyoxylsäure zu. Nach 5 h Rühren unter Rückfluß gießt man das Reaktionsgemisch auf Wasser und extrahiert 3 x mit Ethylacetat. Das Lösungsmittel wird im Vakuum abgezogen und der Rückstand mit Dichlormethan/Methanol (95:5) an $SiO_2$ chromatographiert. Man erhält so 1,2 g (40 % d. Th.) des entsprechenden Azauracils.

Beispiel für Verfahren 4d)

Beispiel 10

2-(4-(2-pyridinyloxy)-3,5-(2H,4H),dioxo-6-cyano-1,2,4-triazin

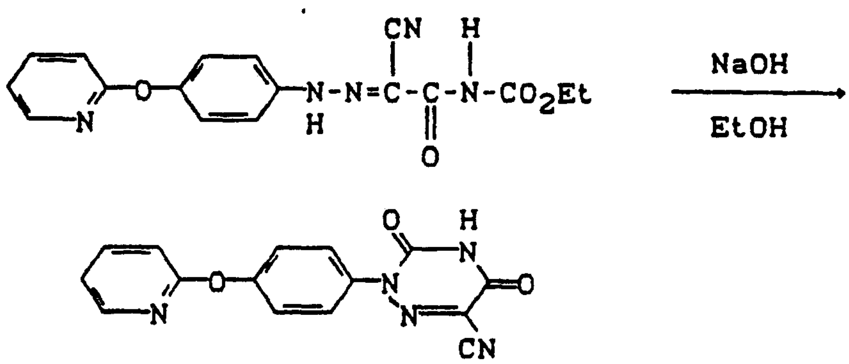


12 g (0,034 mol) des Hydrazonocyanurethans, 1,8 g (0,44 mol) NaOH werden in 50 ml abs. Ethanol 2 h unter Rückfluß erhitzt. Anschließend kühlt man ab, säuert mit Salzsäure an und engt i.V. ein. Man verrührt mit Wasser und saugt den ausgefallenen Niederschlag ab. Man erhält so nach Trocknung 8,9 g (85 % der Theorie) Cyanazauracil.

I Beispiele für Verfahren 4e)

Beispiel 11

2-[4[(4′-Chloro)-2′-thiazolyloxylphenyl]-3,5-(2H,4H)-dioxo-as-triazin

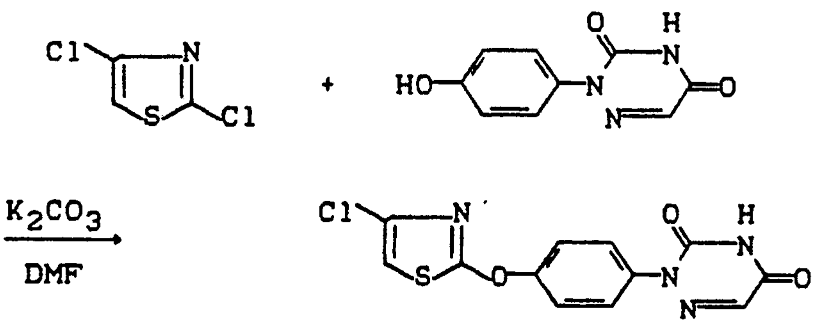


29 g (0,01 mol) Hydroxyphenylazauracil, 1,5 g (0,01 mol) Dichlorthiazol und 1,4 g (0,01 mol) Kaliumcarbonat werden in 20 ml trockenem DMF unter Rückfluß 2 h gerührt. Das abgekühlte Reaktionsgemisch wird mit HCl angesäuert und ausgefallenes Produkt abgesaugt. Nach dem Umkristallisieren aus Ethanol erhält man 2,9 g (90 % der Theorie) Thiazolyloxarylazaurazil.

Beispiel für Verfahren 10a)

Beispiel 12

Ethyl-N-[[[Cyano(4-pyridyloxyphenyl)-hydrazinyliden]-methyl]carbonyl]-carbamt

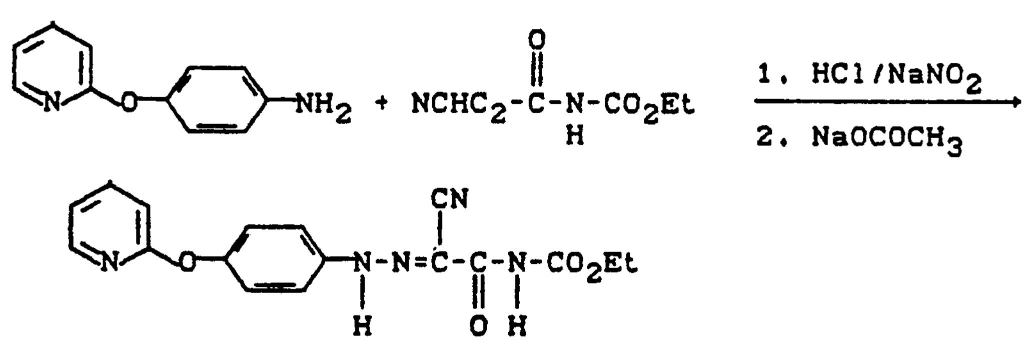


16,9 g (0,091 mol) Pyridyloxyanilin werden in 19,7 ml konz HCl und 200 ml Ethanol gelöst und bei 0-5 °C mit einer Lösung aus 6,4 g (0,092 mol) Natriumnitrit in 30 ml Wasser tropfenweise versetzt. Man rührt bis zur klaren Lösung nach, gibt dann ein Gemisch aus 14,3 g (0,092 mol) Cyanacetylurethan und 21 g (0,25 mol) Natriumacetat zu und läßt 3 h bei 10 °C nachrühren. Das Reaktionsgemisch wird i.V. eingeengt, mit Wasser verrührt und der Feststoff abgesaugt. Man erhält so 25 g (78 %) Produkt als feinkristallines gelbes Pulver.

Beispiel für Verfahren 10b)

Beispiel 13

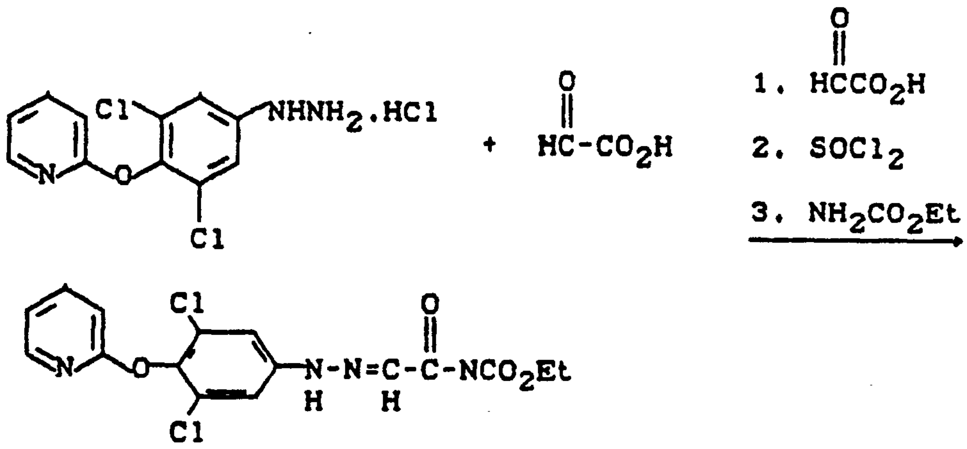


10 g (0,033 mol) Pyridyloxyphenylhydrazinhydrochlorid werden in einem Gemisch aus 150 ml Wasser und 100 ml Ethanol gelöst. Hierzu tropft man 2,9 g (0,039 mol) Glyoxylsäure in 40 ml Wasser. Man rührt 2 h bei Raumtemperatur nach und saugt den Niederschlag ab. Nach Trocknung wird das so gebildete Hydrazon ohne weitere Reinigung in 200 ml Toluol suspendiert und mit 7,8 g (0,066 mol) Thionylchlorid versetzt. Man rührt 1 h unter Rückfluß und destilliert dann das Lösungsmittel im Vakuum ab. Der Rückstand wird in Tetrahydrofuran suspendiert und mit 4,4 g (0,05 mol) Urethan versetzt und 2 h unter Rückfluß gerührt. Nach dem Abziehen des Lösungsmittels wird der Rückstand an $SiO_2$ mit Essigester/Cyclohexan (1:1) chromatographiert. Man erhält so 4,7 g (42 % d. Th.) Hydrazonoverbindung.

Beispiel für Verfahren 12

Beispiel 14

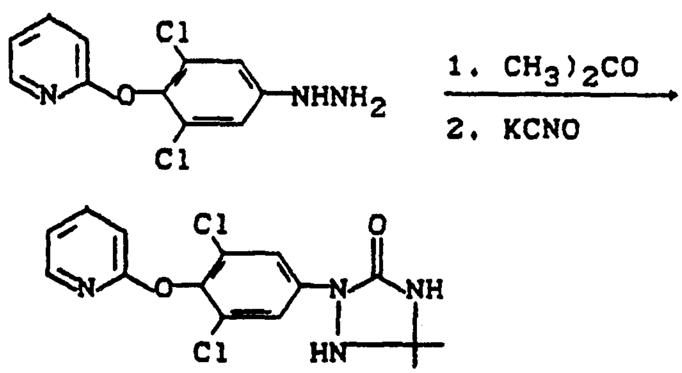


10 g (0,037 mol) Pyridyloxyphenylhydrazin werden in 100 ml Tetrahydrofuran und 30 ml Aceton gelöst. Hierzu tropft man 0,5 ml 20 %ige Schwefelsäure. Man rührt bei Raumtemperatur 3 h nach, engt im Vakuum ein, versetzt mit Wasser und extrahiert 3 x mit Ethylacetat. Trocknen über $Na_2SO_4$ und Abziehen des Lösungsmittels liefern einen Rest öligen Rückstand, der in 50 ml Eisessig gelöst wird. Hierzu gibt man 2 ml Wasser und 3,2 g (0,04 mol) Kaliumcyanat. Man rührt 15 h bei Raumtemperatur, gibt mehrmals 1 g Kaliumcyanat zu und rührt weitere 8 h. Das Reaktionsgemisch wird mit Wasser verdünnt und ausgefallener Feststoff abgesaugt. Man erhält so 5,3 g (44 % d. Th.) 1-[3,5-Dichloro-4-(2-pyridyl)-phenyl]-3,3-dimethyl-1,2,4-triazolidin-5-on.

## Ansprüche

1. Substituierte Hexahydro-1,2,4-triazindione der allgemeinen Formel I

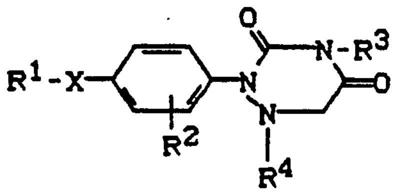

I

in welcher
$R^1$ für über Kohlenstoff gebundene heteroaromatische Reste steht, die gegebenenfalls substituiert sind,
X für O, S, SO, $SO_2$ oder $-CR^5(CN)-$ steht,
$R^2$ für einen oder mehrere, gleiche oder verschiedene Reste der Gruppe Wasserstoff, Halogen, Nitro, Alkyl, Alkoxy, Halogenalkyl, Halogenalkoxy, Alkylthio, Halogenalkylthio,
$R^3$ und $R^4$ unabhängig voneinander für Wasserstoff, gegebenenfalls substituiertes Alkyl, Alkenyl, Alkinyl, Aralkyl stehen,
$R^5$ für Wasserstoff oder Alkyl steht,
ausgenommen Verbindungen, in denen X für $-CR^5(CN)-$und $R^1$ für Thienyl steht.

2. Verfahren zur Herstellung von substituierten Hexahydro-1,2,4-triazindionen der allgemeinen Formel I

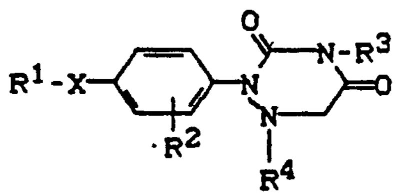

I

in welcher
$R^1$ für über Kohlenstoff gebundene heteroaromatische Reste steht, die gegebenenfalls substituiert sind,
X für O, S, SO, $SO_2$ oder $-CR^5(CN)-$ steht,
$R^2$ für Wasserstoff einen oder mehrere, gleiche oder verschiedene Reste der Gruppe Wasserstoff, Halogen, Nitro, Alkyl, Alkoxy, Halogenalkyl, Halogenalkoxy, Alkylthio, Halogenalkylthio,
$R^3$ und $R^4$ unabhängig voneinander für Wasserstoff, gegebenenfalls substituiertes Alkyl, Alkenyl, Alkinyl, Aralkyl stehen,
$R^5$ für Wasserstoff oder Alkyl steht,
ausgenommen Verbindungen, in denen X für $-CR^5(CN)-$und $R^1$ für Thienyl steht,
a) dadurch gekennzeichnet, daß man Verbindungen der Formel II

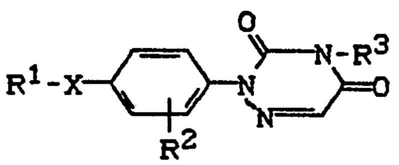

II

in welcher
X, $R^1$, $R^2$ und $R^3$ die oben angegebene Bedeutung haben,
hydriert oder
b) dadurch gekennzeichnet, daß man Verbindungen der Formel III

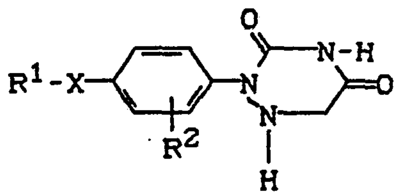

III

in welcher
$R^1$, $R^2$, X die oben angegebene Bedeutung haben mit Verbindungen der Formel IV
$R^3$-B IV
in welcher
$R^3$ für gegebenenfalls substituiertes Alkyl, Alkenyl, Alkinyl, Aralkyl steht und
B für Halogen, -O-$SO_2$-Alkyl, -O-$SO_2$-Aryl, -O-$SO_2$-Halogenalkyl steht
umsetzt, oder
c) dadurch gekennzeichnet, daß man Verbindungen der Formel V

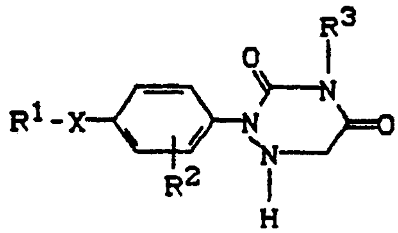

V

in welcher
$R^1$, $R^2$, $R^3$ und X die oben angegebene Bedeutung haben, $R^3$ aber nicht für Wasserstoff steht,
mit Verbindungen der Formel VI
$R^4$-B VI
in welcher
$R^4$ und B die oben angegebene Bedeutung haben,
umsetzt.

3. Verbindungen der Formel II

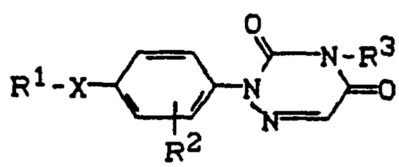

II

in welcher
X die in Anspruch 1 angegebene Bedeutung hat,
$R^1$, $R^2$, $R^3$ die in Anspruch 1 angegebenen Bedeutungen besitzen, wobei $R^1$ nicht für Thiophen stehen darf, wenn X für -C $R^5$(CN) steht und nicht für Benzthiazol, Benzoxazol, Thiazol und Oxazol stehen darf, wenn X für O, S, SO, $SO_2$ steht.

4. Verfahren zur Herstellung der neuen Verbindungen der Formel II gemäß Anspruch 3 sowie bekannter Verbindungen der Formel II, dadurch gekennzeichnet,
a) daß man Verbindungen der Formel VII

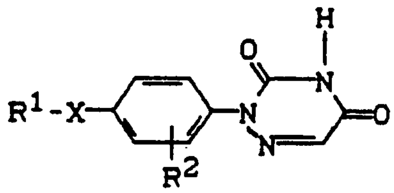 VII

in welcher
X, $R^1$ und $R^2$ die in Anspruch 3 angegebene Bedeutung haben,
mit Verbindungen der Formel IV
$R^3$-B IV
in welcher
$R^3$ und B die in (2) angegebenen Bedeutungen haben,
umsetzt, oder
b) daß man Verbindungen der Formel VIII

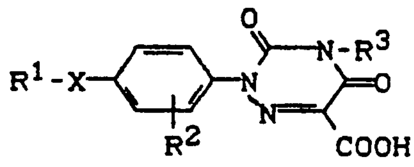 VIII

in welcher
X, $R^1$, $R^2$, $R^3$, $R^5$ die in Anspruch 1 angegebenen deutungen haben,
durch Erhitzen decarboxyliert oder
c) daß man Verbindungen der Formel IX

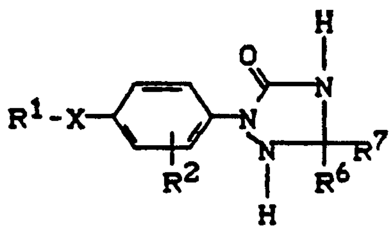 IX

in welcher
X für O, S, -$CR^5$(CN)- steht,
$R^1$, $R^2$ die in Anspruch 1 angegebene Bedeutung haben,
$R^6$ und $R^7$ für gegebenenfalls substituiertes Alkyl stehen,
mit Glyoxylsäure der Formel X
CHO-COOH X
in Gegenwart von anorganischen oder organischen Säuren umsetzt, oder
d) daß man Verbindungen der Formel XI

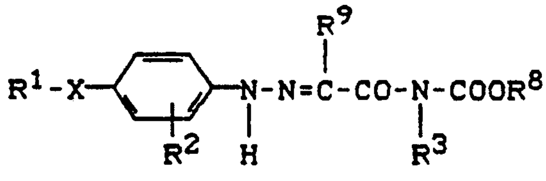 XI

in welcher
X für O, S oder -$CR^5$(CN)- steht,
$R^1$, $R^2$, $R^3$, $R^5$ die in Anspruch 1 angegebene Bedeutung haben, und
$R^8$ für Alkyl oder gegebenenfalls substituiertes Aryl steht,
$R^9$ für H steht,

in Gegenwart von Basen erhitzt.

e) daß man Verbindungen der Formel XVII

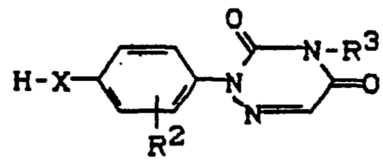

XVII

in welcher

X, $R^2$, $R^3$ die oben angegebene Bedeutung haben,

mit Verbindungen der Formel XVIII

$R^1$-A XVIII

in welcher

$R^1$ die oben angegebene Bedeutung hat und

A für die Reste Halogen, O-$SO_2$-Alkyl, -O-$SO_2$-Halogenalkyl, -O-$SO_2$-Aryl, -S-Alkyl, -$SO_2$-Alkyl oder $SO_2$-Halogenalkyl steht,

umsetzt.

5. Verbindungen der Formel VIII

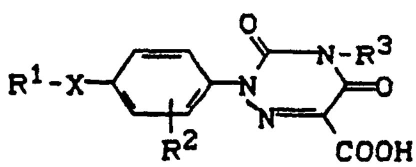

VIII

in welcher

X, $R^1$, $R^2$, $R^3$ die in Anspruch 1 angegebene Bedeutung haben.

6. Verfahren zur Herstellung der neuen Verbindungen der Formel VIII gemäß Anspruch 5, dadurch gekennzeichnet, daß man Verbindungen der Formel XII

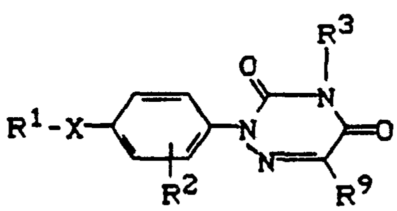

XII

in welcher

X, $R^1$, $R^2$, $R^3$ die in Anspruch 1 angegebene Bedeutung haben,

$R^8$ für Alkyl oder Aryl steht,

$R^9$ für CN oder den Rest -CO-N($R^3$)-COOR$^8$ steht,

in Gegenwart von wäßrigen Mineralsäuren erhitzt.

7. Verbindungen der Formel XII

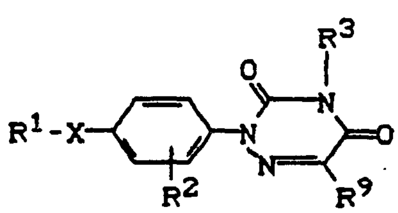

XII

in welcher

X, $R^1$, $R^2$, $R^3$, $R^8$ die in Anspruch 6 angegebene Bedeutung haben, und

$R^9$ für CN oder den Rest -CO-N($R^3$)-COOR$^8$ steht.

8. Verfahren zur Herstellung der neuen Verbindungen der Formel XII gemäß Anspruch 7, dadurch gekennzeichnet, daß man Verbindungen der Formel XI

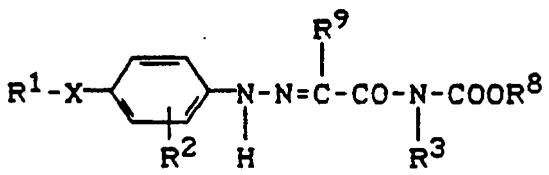

XI

in welcher
X, $R^1$, $R^2$, $R^3$, $R^8$, $R^9$ die in Anspruch 7 angegebene Bedeutung haben,
in Gegenwart von Basen erhitzt.

9. Verbindungen der Formel XI

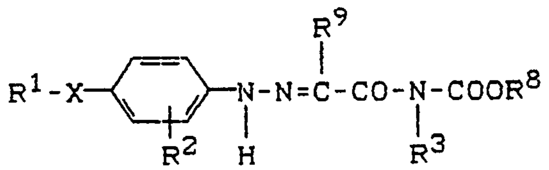

XI

in welcher
X, $R^1$, $R^2$, $R^3$, $R^8$ die in Anspruch 4 angegebene Bedeutung haben
und $R^9$ für H, CN oder den Rest CON($R^3$)-COOR$^8$ steht.

10. Verfahren zur Herstellung der neuen Verbindungen der Formel XI gemäß Anspruch 9, dadurch gekennzeichnet, daß man
a) Verbindungen der Formel XIII

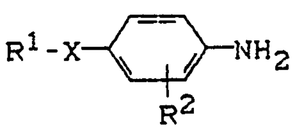

XIII

in welcher
X, $R^1$, $R^2$ die in Anspruch 1 angegebene Bedeutung haben,
in an sich bekannter Weise diazotiert und anschließend mit Verbindungen der Formel XIV

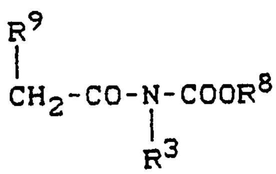

XIV

in welcher
$R^3$, $R^8$, $R^9$ die in Anspruch 7 angegebene Bedeutung haben,
umsetzt, oder
b) indem man Verbindungen der Formel XV

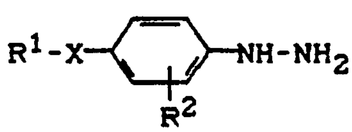

XV

in welcher

X, $R^1$, $R^2$ die in Anspruch 1 angegebene Bedeutung haben, zunächst mit Glyoxylsäure der Formel X, anschließend mit Thionylchlorid und dann mit Ethylurethan umsetzt.

11. Verbindungen der Formel IX

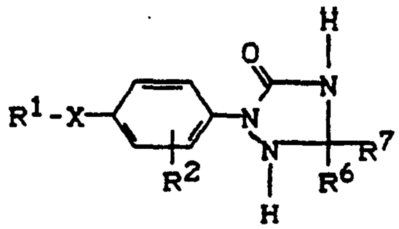

IX

in welcher
X, $R^1$, $R^2$ die in Anspruch 1 angegebene Bedeutung haben,
$R^6$ und $R^7$ unabhängig voneinander für gegebenenfalls substituiertes Alkyl stehen.

12. Verfahren zur Herstellung der neuen Verbindungen der Formel IX gemäß Anspruch 11, dadurch gekennzeichnet, daß man Verbindungen der Formel XV

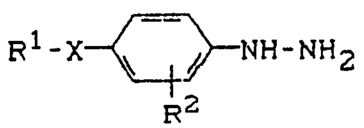

XV

in welcher
X, $R^1$, $R^2$ die in Anspruch 1 angegebene Bedeutung haben,
zunächst mit Ketonen der Formel XVI

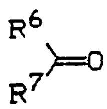

XVI

in welcher
$R^6$ und $R^7$ die in Anspruch 11 angegebene Bedeutung haben,
und anschließend mit Alkalicyanaten umsetzt.

13. Mittel gegen parasitische Protozoen und Fischparasiten, gekennzeichnet durch einen Gehalt an mindestens einem substituierten Hexahydro-1,3,4-triazindion der Formel (I) gemäß Anspruch 1.

14. Verwendung von substituierten Hexahydro-1,3,4-triazindionen der Formel (I) gemäß Anspruch 1 zur Bekämpfung von parasitischen Protozoen und Fischparasiten.

15. Verfahren zur Bekämpfung von parasitischen Protozoen und Fischparasiten, dadurch gekennzeichnet, daß man substituierte Hexahydro-1,3,4-triazindione der Formel (I) gemäß Anspruch 1 auf diese und/oder ihren Lebensraum einwirken läßt.

16. Verfahren zur Herstellung von Mitteln gegen parasitische Protozoen und Fischparasiten, dadurch gekennzeichnet, daß man substituierte Hexahydro-1,3,4-triazindione der Formel (I) gemäß Anspruch 1 mit Streckmitteln und/oder oberflächenaktiven Mitteln vermischt.

17. Verwendung von substituierten Hexahydro-1,3,4-triazindione der Formel (I) gemäß Anspruch 1 zur Herstellung von Mitteln gegen parasitische Protozoen und Fischparasiten.